# EUROPEAN PATENT APPLICATION

(11) **EP 2 471 782 A1**
(43) Date of publication of application: **04.07.2012**
(21) Application number: 10811524.7
(22) Date of filing: 27.08.2010
(51) Int. Cl.: C07D 239/36, A61K 31/513, A61P 3/00, A61P 3/04, A61P 3/10, A61P 9/00, A61P 9/04, A61P 9/10, A61P 9/12, A61P 13/10, A61P 43/00

(54) **NOVEL SULFONAMIDE DERIVATIVE AND PHARMACEUTICAL PRODUCT CONTAINING SAME**

(30) Priority: 27.08.2009 JP 2009197383
(71) Applicant: Kowa Company, Ltd., Nagoya-shi, Aichi 460-8625 (JP)
(72) Inventor: MIURA, Toru, Higashimurayama-shi Tokyo 189-0022 (JP); ONOGI, Kazuhiro, Higashimurayama-shi Tokyo 189-0022 (JP); GOMI, Noriaki, Higashimurayama-shi Tokyo 189-0022 (JP); ARAKI, Takaaki, Higashimurayama-shi Tokyo 189-0022 (JP); TAGASHIRA, Junya, Higashimurayama-shi Tokyo 189-0022 (JP); SEKIMOTO, Ryohei, Higashimurayama-shi Tokyo 189-0022 (JP); ISHIDA, Rie, Higashimurayama-shi Tokyo 189-0022 (JP); AOKI, Hitomi, Higashimurayama-shi Tokyo 189-0022 (JP); OHGIYA, Tadaaki, Higashimurayama-shi Tokyo 189-0022 (JP)
(74) Representative: Adam, Holger
(86) International application number: PCT/JP2010/005286
(87) International publication number: WO 2011/024468

(57) **Abstract**

Disclosed is a novel compound which has both angiotensin II receptor antagonist activity and PPARγ activating activity, and is useful as a prophylactic and/or therapeutic agent for hypertension, heart diseases, angina pectoris, cerebrovascular disorders, cerebral circulatory disorders, ischemic peripheral circulatory disorders, kidney diseases, arteriosclerosis, inflammatory diseases, type 2 diabetes, diabetic complication, insulin resistant syndrome, syndrome X, metabolic syndrome and hyperinsulinemia. Also disclosed is a pharmaceutical composition which contains the novel compound. Specifically disclosed are: a sulfonamide derivative represented by general formula (I), a salt thereof, or a solvate of the derivative or salt; and a pharmaceutical composition which contains the sulfonamide derivative, a salt thereof, or a solvate of the derivative or salt. (In the formula, R¹ represents a C₁₋₆ alkyl group; R² and R³ each represents a C₁₋₆ alkyl group or the like; and R⁴ represents a hydrogen atom, a C₁₋₆ alkyl group, a C₁₋₆ alkanoyl group or a C₃₋₈ cycloalkylcarbonyl group.)

## Description

### TECHNICAL FIELD

The present invention relates to a novel sulfonamide derivative that has both angiotensin II antagonistic activity and PPARγ activation activity, and a pharmaceutical agent containing the same.

### BACKGROUND ART

In recent years, disorders like diabetes, hypertension, dyslipidemia, and obesity which can be a risk factor for arteriosclerotic disorders have been rapidly increasing due to changes in life style with improvements in living standard, i.e., high calorie and high cholesterol type diet, obesity, lack of exercise, aging, and the like. It is known that, although being a risk factor independent of each other, overlap of the disorders can cause an occurrence of arteriosclerotic disorders at higher frequency or aggravation of the disorders. As such, with the understanding of a condition having a plurality of risk factors for arteriosclerotic disorders as metabolic syndrome, efforts have been made to elucidate the cause of the syndrome and to develop a therapeutic method therefor.

Angiotensin II (herein below, it may be also abbreviated as AII) is a peptide that is found to be an intrinsic pressor substance produced by renin-angiotensin system (i.e., RA system). It is believed that pharmacological inhibition of angiotensin II activity can lead to treatment or prevention of circulatory disorders like hypertension. Accordingly, an inhibitor for angiotensin converting enzyme (ACE) which inhibits the enzyme promoting the conversion of angiotensin I (AI) to angiotensin II has been clinically used as an inhibitory agent for RA system. Furthermore, an orally administrable AII receptor blocker (Angiotensin Receptor Blocker: ARB) has been developed, and losartan, candesartan, telmisartan, valsartan, olmesartan, and irbesartan, and the like are already clinically used as a hypotensive agent. It is reported by many clinical or basic studies that, as having not only a hypotensive activity but also other various activities including an anti-inflammatory activity, an endothelial function improving activity, a cardiovascular remodeling inhibiting activity, an oxidation stress inhibiting activity, a proliferation factor inhibiting activity, and insulin resistance improving activity, and the like, ARB is useful for cardiovascular disorders, renal diseases, and arteriosclerosis, and the like (Non-Patent Documents 1 and 2). Most recently, it is also reported that ARB particularly has a kidney protecting activity which does not depend on a hypotensive activity (Non-Patent Document 3).

Meanwhile, three isoforms, i.e., α, γ, and δ, have been identified so far as peroxisome proliferator-activated receptors (PPARs) which belong to a nuclear receptor superfamily. Among them, PPARy is an isoform that is most abundantly expressed in an adipose tissue and it plays an important role in differentiation of adipocytes or metabolism of glycolipids. Currently, thiazolidinedione derivatives (i.e., TZD) like pioglitazone or rosiglitazone are clinically used as a therapeutic agent for diabetes having PPARγ activation activity, and they are known to have an activity of improving insulin resistance, glucose tolerance, and lipid metabolism, and the like. Further, it is recently reported that, based on activation of PPARy, TZD exhibits various activities including a hypotensive activity, an anti-inflammatory activity, an endothelial function improving activity, a proliferation factor inhibiting activity, and an activity of interfering RA system, and the like. It is also reported that, according to such multiple activities, TZD shows a kidney protecting activity particularly in diabetic nephropathy without depending on blood sugar control (Non-Patent Documents 4, 5, 6, 7, and 8). Meanwhile, there is also a concern regarding adverse effects of TZD caused by PPARy activation like body fluid accumulation, body weight gain, peripheral edema, and pulmonary edema (Non-Patent Documents 9 and 10).

It has been recently reported that telmisartan has a PPARy activation activity (Non-Patent Document 11). It has been also reported that the irbesartan has the same activity (Non-Patent Document 12). These compounds have both a RA system inhibiting activity and a PPARy activation activity, and thus are expected to be used as an integrated agent for prevention and/or treatment of circulatory disorders (e.g., hypertension, heart diseases, angina pectoris, cerebrovascular disorders, cerebral circulatory disorders, ischemic peripheral circulatory disorders, and renal diseases, and the like) or diabetes-related disorders (e.g., Type II diabetes, diabetic complications, insulin resistant syndrome, metabolic syndrome, hyperinsulinemia, and the like) without increasing a risk of body fluid accumulation, body weight gain, peripheral edema, pulmonary edema, or congestive heart failure that are concerned over the use of TZD (Patent Document 1). Among them, for diabetic nephropathy, a synergistic prophylactic and/or therapeutic effect is expected from multiple kidney protecting activity based on activities of RA system inhibition and PPARγ activation.

As compounds having such activities, pyrimidine and triazine derivatives (Patent Document 1), imidazopyridine derivatives (Patent Document 2), indole derivatives (Patent Document 3), imidazole derivatives (Patent Document 4), and condensed ring derivatives (Patent Document 5) have been reported.

Among them, in Patent Document 3, a compound represented by the following Formula (A):

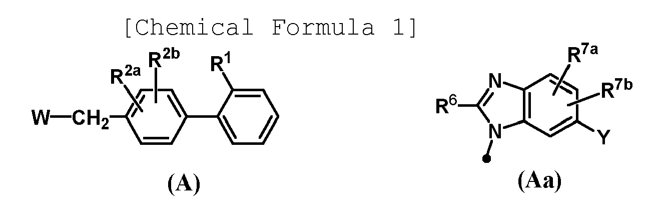

[wherein W represents the Formula (Aa), and R¹ represents a group selected from the following formula:

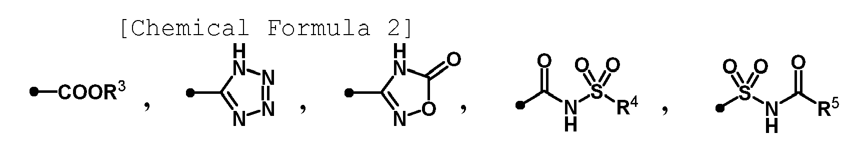

or the like, R^{2a} and R^{2b} represent a hydrogen atom or the like, R³, R⁴, and R⁵ represent a lower alkyl group, R⁶ represents a lower alkyl group or the like, and R^{7a} and R^{7b} represent a hydrogen atom or the like] is disclosed. However, the heterocyclic moieties represented by W are different from those of the compound of the present invention. Furthermore, in Patent Document 3, the biphenyl sulfonamide compounds, which are characteristic compounds of the present invention, are not disclosed in the Examples. Also in other Patent Documents 1, 2, 4, and 5, the biphenyl sulfonamide compounds are neither described nor suggested.
On the other hand, in Patent Document 6, sulfonamide compounds represented by the following formula (B) have been reported:

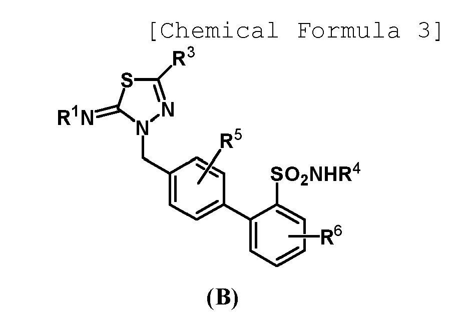

[wherein R¹ represents a hydrogen atom or the like, R³ represents a lower alkyl group or the like, R⁴ represents a hydrogen atom, an acyl group, or the like, and R⁵ and R⁶ represent a hydrogen atom or the like]. In addition, in Patent Document 7, sulfonamide compounds represented by the following Formula (C) have been reported:

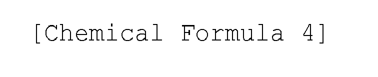

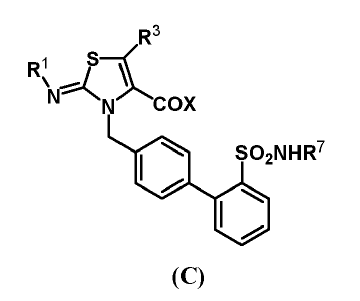

[wherein R¹ represents a hydrogen atom or the like, R³ represents a hydrogen atom, a lower alkyl group, or the like, R⁷ represents a hydrogen atom, a lower alkyl group, an aralkyl group, or an acyl group, and X represents a OH group or the like]. However, the heterocyclic moieties of these compounds are different from those of the compound of the present invention.
In addition, in Patent Document 8, compounds represented by the following Formula (D) have been reported:

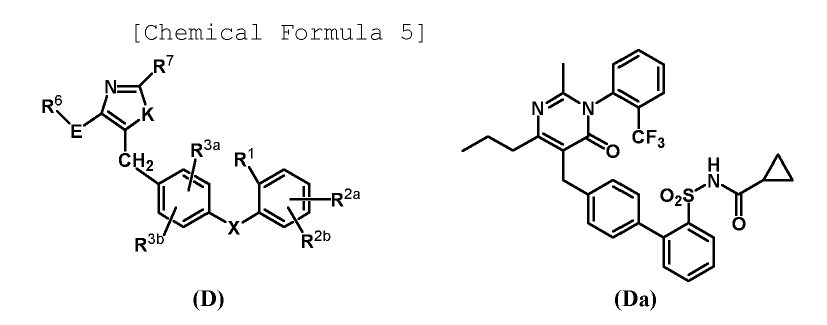

[wherein R¹ represents a carboxyl group, a tetrazolyl group, -SO₂NH-R⁹ (wherein R⁹ represents a hydrogen atom, C₁₋₅ alkyl or the like), -SO₂NH-CO-R²³ (wherein R²³ represents C₃₋₇ cycloalkyl, C₁₋₆ alkyl or the like) or the like, R^{2a}, R^{2b}, R^{3a}, and R^{3b} represent a hydrogen atom or the like, R⁶ represents C₁₋₆ alkyl or the like, R⁷ represents C₁₋₄ alkyl or the like, E and X represents a single bond or a spacer, and K represents -N(R^{8a})-C(=M)- (wherein M represents an oxygen atom or NR²², and R^{8a} and R²² represent an aryl group, a heteroaryl group, or the like) or -N=C(R^{8b}) - (wherein R^{8b} represents a OH group, an aminol group, or the like)]. For example, compounds of the Examples represented by the Formula (Da) are disclosed. Meanwhile, in this document, specific compounds in which a hetero ring is directly-connected to the position of R^{8b} (the position 3 of a pyrimidinone ring) are not disclosed. Furthermore, in Patent Documents 6, 7, and 8, PPARy activation activity as a pharmacological activity, or a treatment for diabetes, obesity or metabolic syndrome is neither described nor suggested.

### PRIOR ART LITERATURE

### PATENT LITERATURE

Patent Document 1: WO 2008/062905
Patent Document 2: WO 2008/084303
Patent Document 3: WO 2008/096820
Patent Document 4: WO 2008/096829
Patent Document 5: WO 2008/143262
Patent Document 6: Japanese Patent Application Laid-Open (JP-A) No. 8-208632
Patent Document 7: JP-A No. 9-20764
Patent Document 8: US Patent No. 5166206

### Non-Patent Document

Non-Patent Document 1: AMER. J. Hypertension, 18, 720 (2005)
Non-Patent Document 2: Current Hypertension Report, 10, 261 (2008)
Non-Patent Document 3: Diabetes Care, 30, 1581 (2007)
Non-Patent Document 4: Kidney Int., 70, 1223 (2006)
Non-Patent Document 5: Circulation, 108, 2941 (2003)
Non-Patent Document 6: Best Pract. Res. Clin. Endocrinol. Metab., 21 (4), 687 (2007)
Non-Patent Document 7: Diab. Vasc. Dis. Res., 1 (2), 76 (2004)
Non-Patent Document 8: Diab. Vasc. Dis. Res., 2 (2), 61 (2005)
Non-Patent Document 9: J. Clin. Invest., 116 (3), 581 (2006)
Non-Patent Document 10: FASEB J., 20 (8), 1203 (2006)
Non-Patent Document 11: Hypertension, 43, 993 (2004)
Non-Patent Document 12: Circulation, 109, 2054 (2004)

### SUMMARY OF THE INVENTION

### PROBLEMS TO BE SOLVED BY THE INVENTION

An object of the present invention is to provide a novel compound that is useful as a pharmaceutical agent for preventing and/or treating hypertension as a circulatory disorder, diabete as a metabolic disease, or the like, and a pharmaceutical composition using the same.

### MEANS FOR SOLVING THE PROBLEMS

As a result of intensive studies to achieve the purpose described above, the inventors of the invention found that the sulfonamide derivative represented by the formula (I) below is a compound that has excellent angiotensin II antagonistic activity and PPARγ activation activity, and therefore completed the invention.

Specifically, the present invention relates to the following inventions.
[1] A sulfonamide derivative represented by the formula (I) below or a salt thereof, or a solvate thereof:

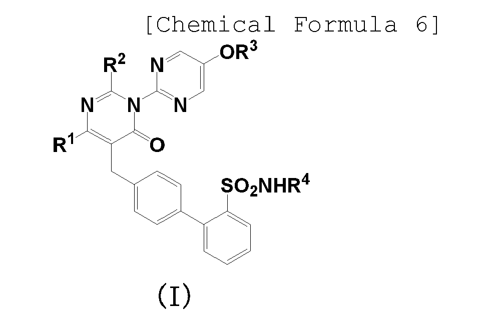

[in the formula, R¹ represents a C₁₋₆ alkyl group,
R² represents a C₁₋₆ alkyl group, a C₃₋₈ cycloalkyl group, or a C₁₋₆ alkyl-pyridinyl-C₁₋₆ alkyl group, R³ represents a hydrogen atom, a C₁₋₆ alkyl group, a halo C₁₋₆ alkyl group, a C₃₋₈ cycloalkyl group, or a C₁₋₆ alkoxy-C₁₋₆ alkyl group, and
R⁴ represents a hydrogen atom, a C₁₋₆ alkyl group, a C₁₋₆ alkanoyl group, or a C₃₋₈ cycloalkylcarbonyl group].

[2] The sulfonamide derivative or the salt thereof, or the solvate thereof described in the above [1], wherein the compound represented by the formula (I) is a compound selected from a group consisting of:
N-{4'-{[4-butyl-1-(5-methoxypyrimidin-2-yl)-2-methyl-6-oxo-1,6-dihydropyrimidin-5-yl]methyl}biphenyl-2-ylsulfony l}propionamide (refer to Example 1),
N-{4'-{[4-butyl-1-(5-methoxypyrimidin-2-yl)-2-methyl-6-oxo-1,6-dihydropyrimidin-5-yl]methyl}biphenyl-2-ylsulfony l}hexanamide (refer to Example 2),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopropanecarboxamide (refer to Example 3),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopentanecarboxamide (refer to Example 4),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclohexanecarboxamide (refer to Example 5),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }propionamide (refer to Example 6),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }hexanamide (refer to Example 7),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopropanecarboxamide (refer to Example 8),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopentanecarboxamide (refer to Example 9),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclohexanecarboxamide (refer to Example 10), N-{4'-{{4-butyl-1-[5-(difluoromethoxy)pyrimidin-2-yl] -2-methyl-6-oxo-1,6-dihydropyrimidin-5-yl}methyl}biphenyl-2 -ylsulfonyl}cyclopropanecarboxamide (refer to Example 11),
N-{4'-{{4-butyl-2-methyl-6-oxo-1-[5-(2,2,2-trifluoroe thoxy)pyrimidin-2-yl]-1,6-dihydropyrimidin-5-yl}methyl}biph enyl-2-ylsulfonyl}cyclopropanecarboxamide (refer to Example 12),
N-{4'-{[4-butyl-1-(5-ethoxypyrimidin-2-yl)-2-ethyl-6-oxo-1,6-dihydropyrimidin-5-yl]methyl}biphenyl-2-ylsulfonyl} cyclopropanecarboxamide (refer to Example 13),
N-{4'-{[4-butyl-1-(5-ethoxypyrimidin-2-yl)-2-isopropy l-6-oxo-1,6-dihydropyrimidin-5-yl]methyl}biphenyl-2-ylsulfo nyl}cyclopropanecarboxamide (refer to Example 14),
N-{4'-{[4-butyl-2-cyclopropyl-1-(5-methoxypyrimidin-2 -yl)-6-oxo-1,6-dihydropyrimidin-5-yl]methyl}biphenyl-2-ylsu lfonyl}cyclopropanecarboxamide (refer to Example 15),
N-{4'-{[4-butyl-2-cyclopropyl-1-(5-ethoxypyrimidin-2-yl)-6-oxo-1,6-dihydropyrimidin-5-yl]methyl}biphenyl-2-ylsul fonyl}cyclopropanecarboxamide (refer to Example 16),
N-{4'-{[4-butyl-2-cyclobutyl-1-(5-ethoxypyrimidin-2-y l)-6-oxo-1,6-dihydropyrimidin-5-yl]methyl}biphenyl-2-ylsulf onyl}cyclopropanecarboxamide (refer to Example 17),
N-{4'-{[4-butyl-2-cyclopentyl-1-(5-ethoxypyrimidin-2-yl)-6-oxo-1,6-dihydropyrimidin-5-yl]methyl}biphenyl-2-ylsul fonyl}cyclopropanecarboxamide (refer to Example 18), and
N-{4'-{[4-butyl-2-cyclohexyl-1-(5-ethoxypyrimidin-2-y l)-6-oxo-1,6-dihydropyrimidin-5-yl]methyl}biphenyl-2-ylsulf onyl}cyclopropanecarboxamide (refer to Example 19).
The alkyl group such as butyl in the nomenclature of the above-mentioned compounds represents a straight (normal) chain unless particularly designated.

[3] A pharmaceutical composition containing: the sulfonamide derivative or the salt thereof, or the solvate thereof described in the above [1] or [2], and a pharmaceutically acceptable carrier.

[4] A pharmaceutical composition containing: the sulfonamide derivative or the salt thereof, or the solvate thereof described in the above [1] or [2] as an effective component, having both angiotensin II receptor antagonistic activity and PPARγ activation activity.

[5] An agent for preventing and/or treating a circulatory disorder containing as an effective component the sulfonamide derivative or the salt thereof, or the solvate thereof described in the above [1] or [2].

[6] The agent for preventing and/or treating a circulatory disease described in the above [5], wherein the circulatory disease is hypertension, heart disease, angina pectoris, cerebral vascular accident, cerebrovascular disorder, ischemic peripheral circulatory disorder, kidney disease, or arteriosclerosis.

[7] An agent for preventing and/or treating a metabolic disease containing as an effective component the sulfonamide derivative or the salt thereof, or the solvate thereof described in the above [1] or [2].

[8] The agent for preventing and/or treating a metabolic disease described in the above [7], wherein the metabolic disease is Type II diabetes mellitus, diabetic complication (diabetic retinopathy, diabetic neuropathy, or diabetic nephropathy), insulin resistant syndrome, metabolic syndrome, or hyperinsulinemia.

[9] A method of preventing and/or treating a circulatory disease **characterized in that** an effective amount of the sulfonamide derivative or the salt thereof, or the solvate thereof described in the above [1] or [2] is administered to a patient in need of the treatment.

[10] A method of preventing and/or treating a metabolic disease **characterized in that** an effective amount of the sulfonamide derivative or the salt thereof, or the solvate thereof described in the above [1] or [2] is administered to a patient in need of the treatment.

[11] Use of the sulfonamide derivative or the salt thereof, or the solvate thereof described in the above [1] or [2] for production of a preparation used for prevention and/or treatment of a circulatory disease.

[12] Use of the sulfonamide derivative or the salt thereof, or the solvate thereof described in the above [1] or [2] for production of a preparation used for prevention and/or treatment of a metabolic disease.

[13] The sulfonamide derivative or the salt thereof, or the solvate thereof described in the above [1] or [2] as a preventive and/or therapeutic agent having both an angiotensin II receptor antagonist activity and a PPARγ activation activity.

### EFFECTS OF THE INVENTION

The sulfonamide derivative represented by the formula (I) of the invention or a salt thereof, or a solvate thereof exhibits a potent antagonistic activity for an angiotensin II receptor, and can be appropriately used as an effective component for an agent for preventing and/or treating a disease related with angiotensin II, for example a circulatory dissease such as hypertension, heart disease, angina pectoris, cerebral vascular accident, cerebrovascular disorder, ischemic peripheral circulatory disorder, kidney disease, and arteriosclerosis.

Further, the sulfonamide derivative represented by the formula (I) of the invention or a salt thereof, or a solvate thereof has a PPARγ activation activity and can be appropriately used as an effective component for an agent for preventing and/or treating a disease related with PPARγ, for example metabolic disease such as arterosclerosis, Type II diabetes mellitus, diabetic complication (diabetic retinopathy, diabetic neuropathy, or diabetic nephropathy), insulin resistance syndrome, syndrome X, metabolic syndrome, and hyperinsulinemia.

Still further, the sulfonamide derivative represented by the formula (I) of the invention, or a salt thereof, or a solvate thereof has both an antagonistic activity for an angiotensin II receptor and PPARγ activation activity and can be appropriately used as an effective component for an agent for preventing and/or treating a disease related with both angiotensin II and PPARγ, for example, arterosclerosis, diabetic nephropathy, insulin resistance syndrome, syndrome X, and metabolic syndrome.

### MODES FOR CARRYING OUT THE INVENTION

The "halogen atom" as used herein includes a fluorine atom, a chlorine atom, a bromine atom, an iodine atom, and the like.

The "C₁₋₆ alkyl group" and the "C₁₋₆ alkyl" as used herein mean a linear or branched hydrocarbon group having 1 to 6 carbon atoms, and examples thereof include a methyl group, an ethyl group, a n-propyl group, an isopropyl group, a n-butyl group, an isobutyl group, a t-butyl group, a n-pentyl group, a 2-methylbutyl group, a 2,2-dimethylpropyl group, a n-hexyl group, and the like.

The "C₃₋₈ cycloalkyl group" and the "C₃₋₈ cycloalkyl" as used herein include saturated or unsaturated monocyclic, polycyclic, or condensed cyclic cycloalkyl group having 3 to 8 carbon atoms, and preferably 3 to 6 carbon atoms. Examples of such cycloalkyl group include a cyclopropyl group, a cyclobutyl group, a cyclopentyl group, a cyclohexyl group, a cyclopentyl group, and a cyclooctyl group.

The "C₁₋₆ alkyl-pyridinyl-C₁₋₆ alkyl group" as used herein means a C₁₋₆ alkyl group substituted with a pyridinyl group that is substituted with one to four C₁₋₆ alkyls. The substitution position in the pyridine ring is not particularly limited, but an alkyl-substituted pyridin-2-yl-alkyl group is preferable. Specifically, examples thereof include, a 3-methylpyridin-2-ylmethyl group, a 3-ethylpyridin-2-ylmethyl group, a 3-methylpyridin-2-ylethyl group, a 3-ethylpyridin-2-ylethyl group, a 4-ethylpyridin-2-ylethyl group, a 5-ethylpyridin-2-ylethyl group, a 6-ethylpyridin-2-ylethyl group, a 3,4-dimethylpyridin-2-ylethyl group, a 3,5-dimethylpyridin-2-ylethyl group, and the like.

The "halo C₁₋₆ alkyl group" and the "halo C₁₋₆ alkyl" as used herein mean a linear or a branched alkyl group having 1 to 6 carbon atoms substituted with one to the largest possible number of halogen atoms, and examples thereof include a fluoromethyl group, a difluoromethyl group, a trifluoromethyl group, a 2,2,2-trifluoroethyl group, a 1,1,2,2,2-pentafluoroethyl group, a 3,3,3-trifluoropropyl group, and the like.

The "C₁₋₆ alkoxy-C₁₋₆ alkyl group" as used herein means a C₁₋₆ alkyl group that is substituted with one to three C₁₋₆ alkoxys. Specifically, examples thereof include a methoxymethyl group, an ethoxymethyl group, an ethoxyethyl group, a propoxymethyl group, a propoxyethyl group, a butoxymethyl group, a butoxyethyl group, a 2,2-dimethoxyethyl group, and the like.

The "C₁₋₆ alkanoyl group", and the "C₁₋₆ alkanoyl"as used herein mean a linear or branched alkanoyl group having 1 to 6 carbon atoms, and examples thereof include a formyl group, an acetyl group, a propionyl group, a butyryl group, a valeryl group, an isovaleryl group, a pivaloyl group, a hexanoyl group, and the like.

Examples of the "C₃₋₈ cycloalkylcarbonyl group" and the "C₃₋₈ cycloalkylcarbonyl" as used herein include groups in which saturated cycloalkyl having 3 to 8 carbon atoms, and preferably 3 to 6 carbon atoms, is bonded to one side of the carbonyl group. Examples of such cycloalkylcarbonyl group include, a cyclopropylcarbonyl group, a cyclobutylcarbonyl group, a cyclopentylcarbonyl group, a cyclohexylcarbonyl group, a cycloheptylcarbonyl group, a cyclooctylcarbonyl group, and the like.

Examples of the preferred mode of the invention include the followings.

As for the R¹ of the formula (I), preferred examples of the C₁₋₆ alkyl group include a C₁₋₄ alkyl group, and more preferred examples include a C₂₋₄ alkyl group. For example, an ethyl group, a n-propyl group and a n-butyl group are preferable.

As for R² in the formula (I), preferred examples of the C₁₋₆ alkyl group include C₁₋₄ alkyl group. For example, a methyl group is preferable.

As for R² in the formula (I), preferred examples of the C₃₋₈ cycloalkyl group include C₃₋₆ cycloalkyl. For example, a cyclopropyl group, a cyclobutyl group, and a cyclopentyl group are preferable.

As for R² in the formula (I), preferred examples of the "C₁₋₆ alkyl-pyridinyl-C₁₋₆ alkyl group" include a "C₁₋₄ alkyl-pyridinyl-C₁₋₄ alkyl group", and more preferred examples include the "C₁₋₄ alkyl-pyridin-2-yl-C₁₋₄ alkyl group". For example, a 5-ethylpyridin-2-ylethyl group is preferable.

As for R³ in the formula (I), preferred examples of the C₁₋₆ alkyl group include a C₁₋₄ alkyl group. For example, a methyl group and an ethyl group are preferable.

As for R³ in the formula (I), preferred examples of the halo C₁₋₆ alkyl group include a halo C₁₋₄ alkyl group. For example, a difluoromethyl group, a trifluoromethyl group, and a 2,2,2-trifluoroethyl group are preferable.

As for R³ in the formula (I), preferred examples of the C₃₋₈ cycloalkyl group include a C₃₋₆ cycloalkyl group. For example, a cyclopropyl group, a cyclobutyl group, and a cyclopentyl group are preferable.

As for R³ in the formula (I), preferred examples of the "C₁₋₆ alkoxy-C₁₋₆ alkyl group" include a "C₁₋₄ alkoxy-C₁₋₄ alkyl group". For example, a methoxyethyl group is preferable.

As for R⁴ in the formula (I), preferred examples of the C₁₋₆ alkyl group include a C₁₋₄ alkyl group, and more preferred examples include a C₂₋₄ alkyl group. For example, an ethyl group, a n-propyl group, and a t-butyl group are preferable.

As for R⁴ in the formula (I), preferred examples of the C₁₋₆ alkanoyl group include a C₁₋₅ alkanoyl group, and more preferred examples include a C₁₋₄ alkanoyl group. For example, a propionyl group, a butyryl group, and a valeryl group are preferable.

As for R⁴ in the general formula (I), preferred examples of the C₃₋₈ cycloalkylcarbonyl group include a group in which a C₃₋₆ cycloalkyl group is bonded to a carbonyl group. For example, a cyclopropylcarbonyl group, a cyclobutylcarbonyl group, and a cyclopentylcarbonyl group are preferable.

More preferred examples of the sulfonamide derivative compounds that are represented by the formula (I) include a compound that is selected from a group consisting of following compounds:
N-{4'-{[4-butyl-1-(5-methoxypyrimidin-2-yl)-2-methyl-6-oxo-1,6-dihydropyrimidin-5-yl]methyl}biphenyl-2-ylsulfony l}propionamide (refer to Example 1),
N-{4'-{[4-butyl-1-(5-methoxypyrimidin-2-yl)-2-methyl-6-oxo-1,6-dihydropyrimidin-5-yl]methyl}biphenyl-2-ylsulfony l}hexanamide (refer to Example 2),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopropanecarboxamide (refer to Example 3),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopentanecarboxamide (refer to Example 4),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclohexanecarboxamide (refer to Example 5),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }propionamide (refer to Example 6),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }hexanamide (refer to Example 7),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopropanecarboxamide (refer to Example 8),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopentanecarboxamide (refer to Example 9),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclohexanecarboxamide (refer to Example 10),
N-{4'-{{4-butyl-1-[5-(difluoromethoxy)pyrimidin-2-yl] -2-methyl-6-oxo-1,6-dihydropyrimidin-5-yl}methyl}biphenyl-2 -ylsulfonyl}cyclopropanecarboxamide (refer to Example 11),
N-{4'-{{4-butyl-2-methyl-6-oxo-1-[5-(2,2,2-trifluoroe thoxy)pyrimidin-2-yl]-1,6-dihydropyrimidin-5-yl}methyl}biph enyl-2-ylsulfonyl}cyclopropanecarboxamide (refer to Example 12),
N-{4'-{[4-butyl-1-(5-ethoxypyrimidin-2-yl)-2-ethyl-6-oxo-1,6-dihydropyrimidin-5-yl]methyl}biphenyl-2-ylsulfonyl} cyclopropanecarboxamide (refer to Example 13),
N-{4'-{[4-butyl-1-(5-ethoxypyrimidin-2-yl)-2-isopropy l-6-oxo-1,6-dihydropyrimidin-5-yl]methyl}biphenyl-2-ylsulfo nyl}cyclopropanecarboxamide (refer to Example 14),
N-{4'-{[4-butyl--2-cyclopropyl-1-(5-methoxypyrimidin-2-yl)-6-oxo-1,6-dihydropyrimidin-5-yl]methyl}biphenyl-2-yls ulfonyl}cyclopropanecarboxamide (refer to Example 15),
N-{4'-{[4-butyl-2-cyclopropyl-1-(5-ethoxypyrimidin-2-yl)-6-oxo-1,6-dihydropyrimidin-5-yl]methyl}biphenyl-2-ylsul fonyl}cyclopropanecarboxamide (refer to Example 16),
N-{4'-{[4-butyl-2-cyclobutyl-1-(5-ethoxypyrimidin-2-y l)-6-oxo-1,6-dihydropyrimidin-5-yl]methyl}biphenyl-2-ylsulf onyl}cyclopropanecarboxamide (refer to Example 17),
N-{4'-{[4-butyl-2-cyclopentyl-1-(5-ethoxypyrimidin-2-yl)-6-oxo-1,6-dihydropyrimidin-5-yl]methyl}biphenyl-2-ylsul fonyl}cyclopropanecarboxamide (refer to Example 18), and
N-{4'-{[4-butyl-2-cyclohexyl-1-(5-ethoxypyrimidin-2-y l)-6-oxo-1,6-dihydropyrimidin-5-yl]methyl}biphenyl-2-ylsulf onyl}cyclopropanecarboxamide (refer to Example 19).

Still more preferred examples of the sulfonamide derivatives that are represented by the formula (I) include a compound that is selected from a group consisting of following compounds:
N-{4'-{[4-butyl-1-(5-methoxypyrimidin-2-yl)-2-methyl-6-oxo-1,6-dihydropyrimidin-5-yl]methyl}biphenyl-2-ylsulfony l}propionamide (refer to Example 1),
N-{4'-{[4-butyl-1-(5-methoxypyrimidin-2-yl)-2-methyl-6-oxo-1,6-dihydropyrimidin-5-yl]methyl}biphenyl-2-ylsulfony l}hexanamide (refer to Example 2),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopropanecarboxamide (refer to Example 3),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopentanecarboxamide (refer to Example 4),
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclohexanecarboxamide (refer to Example 5),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }hexanamide (refer to Example 7),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopropanecarboxamide (refer to Example 8),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopentanecarboxamide (refer to Example 9),
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclohexanecarboxamide (refer to Example 10), and
N-{4'-{[4-butyl-2-cyclopropyl-1-(5-ethoxypyrimidin-2-yl)-6-oxo-1,6-dihydropyrimidin-5-yl]methyl}biphenyl-2-ylsul fonyl}cyclopropanecarboxamide (refer to Example 16).

If the compound of the invention has geometrical isomers or optical isomers, the invention encompasses all of such insomers. Isolation of these isomers is carried out by an ordinary method.

Salts of the compound represented by the formula (I) are not particularly limited, if they are pharmaceutically acceptable salts. When the compound is processed as an acidic compound, an alkali metal salt or an alkali earth metal salt like sodium salt, potassium salt, magnesium salt, and calcium salt; and a salt with an organic base like trimethylamine, triethylamine, pyridine, picoline, N-methylpyrrolidine, N-methylpiperidine, and N-methylmorpholine can be mentioned. When the compound is processed as a basic compound, an acid addition salt and the like including a salt with a mineral acid, for example, hydrochloric acid salt, hydrobromic acid salt, hydriodic acid salt, sulfuric acid salt, nitric acid salt, phosphoric acid salt and the like; an organic acid addition salt, for example, benzoic acid salt, methanesulfonic acid salt, ethanesulfonic acid salt, benzene sulfonic acid salt, p-toluene sulfonic acid salt, maleic acid salt, fumaric acid salt, tartaric acid salt, citric acid salt, and acetic acid salt; or the like can be mentioned.

Examples of the solvate of the compound represented by the formula (I) or salt thereof include a hydrate and the like, but not limited thereto.

In addition, compounds which are metabolized in a living body and converted into the compounds represented by the aforementioned formula (I), so called prodrugs, all fall within the scope of the compounds of the invention. Examples of groups which form the prodrugs of the compounds of the invention include the groups described in "Progress in Medicine", vol. 5, pp. 2157-2161, 1985, Life Science Medica, and the groups described in "Development of Drugs", vol. 7, Molecular Designs, pp. 163 to 198, 1990, Hirokawa Shoten.

The compounds represented by the formula (I), or salts or solvates thereof can be produced according to various known methods, and the production method is not specifically limited. For example, the compounds can be produced according to the following reaction step. Further, when each reaction shown below is performed, functional groups other than the reaction sites may be protected beforehand as required, and deprotected in an appropriate stage. Furthermore, the reaction in each step may be performed by an ordinarily used method, and isolation and purification can be performed by a method suitably selected from conventional methods such as crystallization, recrystallization, chromatography, or the like, or a combination thereof.

### Method for production of the compound represented by the formula (I), or a salt thereof, or a solvate thereof

The compounds represented by the formula (I) of the invention can be produced according to the following method, but the production method is not limited thereto.

Specifically, as illustrated in the Reaction pathway 1 below, oxocarboxylic acid ester represented by the formula (II) is reacted with ammonium acetate, and subsequently reacted with acid anhydride represented by the formula (III) or acid chloride represented by the formula (IV) to give an acylamino compound represented by the formula (V). The acylamino compound represented by the formula (V) is reacted with an aminopyrimidine compound represented by the formula (VI) to give a pyrimidinone derivative represented by the formula (VII). The pyrimidinone derivative represented by the formula (VII) is reacted with a boronic acid compound represented by the formula (VIII) to give a sulfonamide derivative (IX). The sulfonamide derivative represented by the formula (IX) is deprotected, and then reacted with halide represented by the formula (XI) to produce the compound represented by the formula (I) of the invention.

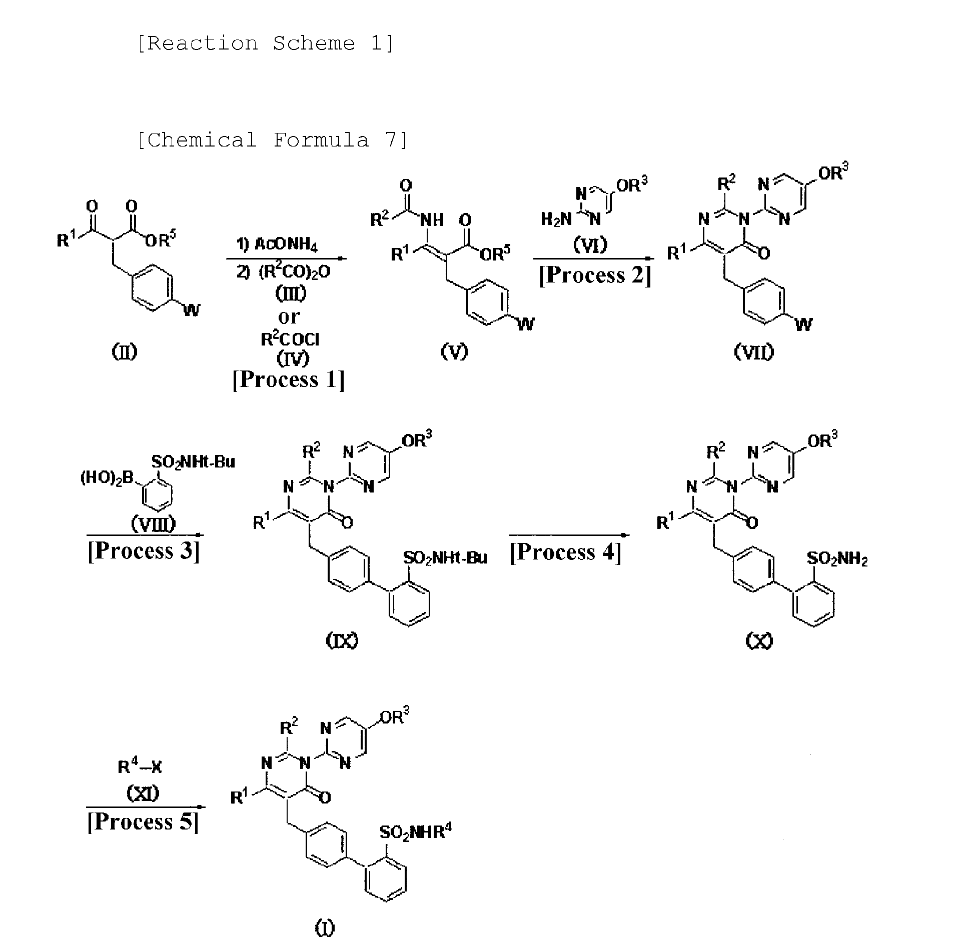

(in the formula, R¹, R², R³, and R⁴ are as defined above, R⁵ represents a protecting group for the carboxyl group, and W represents a leaving group such as a halogen atom.)

### [Process 1-1]

The reaction between the oxocarboxylic acid ester (II) and ammonium acetate may be carried out in a solvent in the presence of an acid. The solvent is not specifically limited, and methanol, ethanol, isopropanol, ethyl acetate, isopropyl acetate, toluene, benzene, dioxane, tetrahydrofuran, acetonitrile, propionitrile, N,N-dimethylformamide, N-methylpyrrolidone, dimethylsulfoxide, and the like may be used either alone or in combination thereof. The acid is not particularly limited, and the examples thereof include a protonic acid like acetic acid, trifluoroacetic acid, propionic acid, benzoic acid, and the like and Lewis acid like titanium tetrachloride, boron trifluoride, stannic chloride, and the like. The reaction conditions may vary depending on the reaction materials used. However, the reaction is generally carried out at 0 to 180°C, preferably 50°C to 150°C for 1 minute to 24 hours, and more preferably 5 minutes to 18 hours.

### [Process 1-2]

The reaction between the crude product obtained by distilling the solvent away and acid anhydride (III) may be carried out in the presence of an acid. The acid is not particularly limited, and examples thereof include a protonic acid like acetic acid, trifluoroacetic acid, propionic acid, benzoic acid, and the like. The reaction conditions may vary depending on the reaction materials used. However, the reaction is generally carried out at 0 to 180°C, preferably 50°C to 120°C for 1 minute to 2 days, and more preferably 5 minutes to 24 hours to obtain the acylamino compound (V).

In addition, the reaction between the crude product obtained by distilling the solvent away and acid chloride (IV) may be carried out in a solvent, in the presence or in the absence of a base. The solvent is not specifically limited, and tetrahydrofuran, toluene, dioxane, N,N-dimethylformamide, N-methylpyrrolidone, dichloromethane, chloroform, acetonitrile, propionitrile, and the like may be used either alone or in combination thereof. The base is not particularly limited, and the examples thereof include organic bases like pyridine, N,N-dimethylaminopyridine (DMAP), collidine, lutidine, 1,8-diazabicyclo[5.4.0]-7-undecene (DBU), 1,5-diazabicyclo[4.3.0]-5-nonene (DBN), 1,4-diazabicyclo[2.2.2]octane (DABCO), triethylamine, diisopropyl amine, diisopropyl ethyl amine, diisopropyl pentyl amine, trimethyl amine, and the like, alkali metal hydrides like lithium hydride, sodium hydride, potassium hydride, and the like, alkali metal hydroxides like lithium hydroxide, sodium hydroxide, potassium hydroxide, and the like, alkali metal carbonates like lithium carbonate, sodium carbonate, potassium carbonate, cesium carbonate, and the like, sodium hydrocarbonate, and the like. The reaction conditions may vary depending on the reaction materials used. However, the reaction is carried out generally at -20 to 100°C, preferably 15 to 80°C for 5 minutes to 48 hours, and preferably 5 hours to 36 hours to obtain the acylamino compound (V).

### [Process 2]

The reaction between the acylamino compound (V) obtained in the method described above and an aminopyrimidine compound (VI) may be carried out in a solvent, in the presence of trialkyl aluminum. The solvent is not specifically limited, and 1,2-dichloroethane, chloroform, dichloromethane, ethyl acetate, isopropyl acetate, toluene, benzene, tetrahydrofuran, dioxane, acetonitrile, propionitrile, hexane, and the like may be used either alone or in combination thereof. As the trialkyl aluminum, the examples thereof include trimethylaluminum, triethylaluminum, tripropylaluminum, and the like. The reaction conditions may vary depending on the reaction materials used. However, the reaction is carried out generally at 0 to 150°C, preferably at 50°C to 120°C for 1 minute to 24 hours, and more preferably 5 minutes to 20 hours to obtain the pyrimidinone derivative (VII).

### [Process 3]

The reaction between the pyrimidinone derivative (VII) obtained in the method described above and a boronic acid compound (VIII) may be carried out in a solvent in the presence of a base and a palladium complex. The solvent is not specifically limited, and benzene, toluene, ether, tetrahydrofuran, dioxane, acetonitrile, N,N-dimethylformamide, ethanol, methanol, propanol, dimethoxyethane, water, and the like may be used either alone or in combination thereof. The base is not specifically limited, and the reaction is carried out preferably under a basic condition by addition of, for example, a non-nucleophilic tertiary amine like triethylamine and diisopropyl ethyl amine, an inorganic base like potassium carbonate, sodium carbonate, cesium carbonate, thallium carbonate, potassium hydroxide, sodium hydroxide, thallium hydroxide, potassium phosphate, sodium phosphate and the like, or an alkoxides of these alkali metals. When an inorganic base that is insoluble in an organic solvent is used, it needs to be used as an aqueous solution, and the reaction is preferably carried out in the presence of a phase-transfer catalyst like tetra-n-butyl ammonium bromide, crown ether, and the like. The palladium complex used in this reaction is not specifically limited, and the examples thereof include tetrakis (triphenylphosphine) palladium, bis(dibenzylideneacetone) palladium, tris(dibenzylideneacetone) palladium or a divalent palladium phosphine complex, and the like. Examples of the divalent palladium phosphine complex include bis(triphenylphosphine) palladium chloride, bis (triphenylphosphine) palladium bromide, bis(triphenylphosphine) palladium acetate, bis(triisopropyl phosphite) palladium chloride, bis(triisopropyl phosphite) palladium bromide, bis(triisopropyl phosphite) palladium acetate, and the like. The reaction conditions may vary depending on the reaction materials used. However, the reaction is carried out generally at 0 to 150°C, preferably 50°C to 120°C for 1 minute to 24 hours, and more preferably 5 minutes to 20 hours to obtain the sulfonamide derivative (IX).

### [Process 4]

Deprotection of the tertiary butyl group of the sulfonamide derivative (IX) obtained in the method described above may be carried out in a solvent or without solvent, in the presence or in the absence of an additive and in the presence of an acid. The solvent is not specifically limited, and methanol, ethanol, isopropanol, ethyl acetate, isopropyl acetate, toluene, benzene, dioxane, tetrahydrofuran, acetonitrile, propionitrile, N,N-dimethylformamide, N-methylpyrrolidone, dimethyl sulfoxide, and the like may be used either alone or in combination thereof. The additive is not specifically limited and the examples thereof include anisole, thioanisole, and the like. The acid is not specifically limited, and the examples thereof include a protonic acid like acetic acid, trifluoroacetic acid, propionic acid, benzoic acid, and the like and a Lewis acid like titanium tetrachloride, boron trifluoride, stannic chloride, and the like. The reaction conditions may vary depending on the reaction materials used. However, the reaction is carried out generally at 0 to 150°C, preferably 20°C to 100°C for 1 minute to 2 days, and more preferably 5 minutes to 36 hours to obtain the sulfonamide derivative (X).

### [Process 5]

The reaction between the sulfonamide derivative (X) obtained in the method described above and halide (XI) may be carried out in a solvent in the presence or in the absence of a base. The solvent is not specifically limited, and tetrahydrofuran, toluene, dioxane, N,N-dimethylformamide, N-methylpyrrolidone, dichloromethane, chloroform, acetonitrile, propionitrile, and the like may be used either alone or in combination thereof. The base is not specifically limited, and the examples thereof include organic bases like pyridine, N,N-dimethyl aminopyridine (DMAP), collidine, lutidine, 1,8-diazabicyclo[5.4.0]-7-undecene (DBU), 1,5-diazabicyclo[4.3.0]-5-nonene (DBN), 1,4-diazabicyclo[2.2.2]octane (DABCO), triethylamine, diisopropylamine, diisopropylethylamine, diisopropylpentylamine, trimethylamine, and the like, alkali metal hydrides like lithium hydride, sodium hydride, potassium hydride, and the like, alkali metal hydroxides like lithium hydroxide, sodium hydroxide, potassium hydroxide, and the like, alkali metal carbonates like lithium carbonate, sodium carbonate, potassium carbonate, cesium carbonate, and the like, sodium hydrocarbonate, and the like. The reaction conditions may vary depending on the reaction materials used. However, the reaction is carried out generally at -20 to 100°C, preferably 15 to 80°C for 5 minutes to 36 hours, and preferably 5 hours to 24 hours to obtain the compound (I).

Further, the pyrimidinone derivative (VII) may be also produced according to the method given below, but it is not limited thereto.

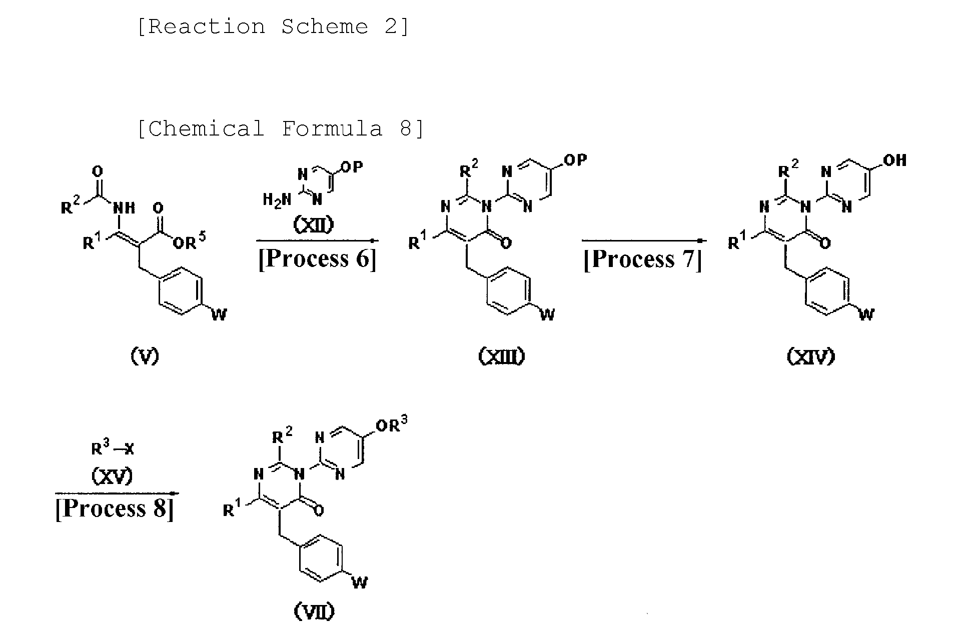

(in the formula, R¹, R², and R³ are as defined above, R⁵ represents a protecting group for the carboxyl group, W represents a leaving group like a halogen atom, and P represents a protecting group for the hydroxide group.)

Specifically, as illustrated in the following Reaction pathway 2, an acylamino compound (V) and an aminopyrimidine compound (XII) are reacted to give a pyrimidinone derivative (XIII). The pyrimidinone derivative (XIII) is deprotected, and then reacted with a halide (XV) to give a pyrimidinone derivative (VII).

### [Process 6]

The reaction between the acylamino compound (V) and the aminopyrimidine compound (XII) may be carried out in a solvent in the presence of trialkyl aluminum. The solvent is not specifically limited, and 1,2-dichloroethane, chloroform, dichloromethane, ethyl acetate, isopropyl acetate, toluene, benzene, tetrahydrofuran, dioxane, acetonitrile, propionitrile, hexane, and the like may be used either alone or in combination thereof. As the trialkyl aluminum, the examples thereof include trimethylaluminum, triethylaluminum, tripropylaluminum, and the like. The reaction conditions may vary depending on the reaction materials used. However, the reaction is carried out generally at 0°C to 150°C, preferably 50°C to 120°C for 1 minute to 24 hours, more preferably 5 minutes to 20 hours to obtain the pyrimidinone derivative (XIII).

### [Process 7]

Deprotection of the protecting group P of the pyrimidinone derivative (XIII) obtained in the method described above is not specifically limited, and it can be carried out with reference to the method that is generally used as deprotection conditions of the protecting group (Protecting groups in Organic Synthesis Fourth Edition, John Wiley & Sons, Inc.) to obtain the compound (XIV).

### [Process 8]

The reaction between the compound (XIV) and a halide (XV) may be carried out in a solvent in the presence or in the absence of a base. The solvent is not specifically limited, and tetrahydrofuran, toluene, dioxane, N,N-dimethylformamide, N-methylpyrrolidone, dichloromethane, chloroform, acetonitrile, propionitrile, and the like may be used either alone or in combination thereof. The base is not specifically limited, and organic bases like pyridine, N,N-dimethyl aminopyridine (DMAP), collidine, lutidine, 1,8-diazabicyclo[5.4.0]-7-undecene (DBU), 1,5-diazabicyclo[4.3.0]-5-nonene (DBN), 1,4-diazabicyclo[2.2.2]octane (DABCO), triethylamine, diisopropylamine, diisopropylethylamine, diisopropylpentylamine, trimethylamine, and the like, alkali metal hydrides like lithium hydride, sodium hydride, potassium hydride, and the like, alkali metal hydroxides like lithium hydroxide, sodium hydroxide, potassium hydroxide, and the like, alkali metal carbonates like lithium carbonate, sodium carbonate, potassium carbonate, cesium carbonate and the like, sodium hydrocarbonate, and the like. The reaction conditions may vary depending on the reaction materials used. However, the reaction is carried out generally at -20 to 100°C, preferably 15 to 80°C for 5 minutes to 36 hours, preferably 5 hours to 24 hours to obtain the pyrimidinone derivative (VII).

If necessary, the intermediates and target compounds that are obtained from each of the reaction above can be isolated and purified by a purification method that is generally used in a field of organic synthesis chemistry, e.g., filtration, extraction,washing, drying, concentration, recrystallization, various chromatographic methods, and the like. Furthermore, the intermediates may be used for the next reaction without any specific purification.

Various isomers may be isolated by applying a general method based on a difference in physicochemical properties among the isomers. For example, a racemic mixture may be resolved into an optically pure isomer by common racemic resolution like optical resolution by which a diastereomer salt is formed with a common optically active acid like tartaric acid or a method of using optically active chromatography. Further, a mixture of diastereomers can be resolved by fractional crystallization or various chromatographic methods, for example. Furthermore, an optically active compound can be also produced by using an appropriate starting compound that is optically active.

The compound (I) obtained may be converted into a salt according to a common method. Furthermore, it may be converted into a solvate with a solvent like a solvent for reaction or a solvent for recrystallization, or into a hydrate.

Examples of a dosage form of the pharmaceutical composition containing the compounds of the invention, salts or solvates thereof as an effective component include, for example, those for oral administration such as tablet, capsule, granule, powder, syrup, or the like and those for parenteral administration such as intravenous injection, intramuscular injection, suppository, inhalant, transdermal preparation, eye drop, nasal drop, or the like. In order to prepare a pharmaceutical preparation in the various dosage forms, the effective component may be used alone, or may be used in appropriate combination with other pharmaceutically acceptable carriers such as excipients, binders, extending agents, disintegrating agents, surfactants, lubricants, dispersing agents, buffering agents, preservatives, corrigents, perfumes, coating agents, diluents, and the like to give a pharmaceutical composition.

Although the administration amount of the pharmaceutical agent of the invention may vary depending on the weight, age, sex, symptoms, and the like of a patient, in terms of the compound represented by the general formula (I), generally 0.1 to 1000 mg, especially 1 to 300 mg, may be administered orally or parenterally at one time or several times as divided portions per day for an adult.

### EXAMPLES

Hereinbelow, the invention will be explained in greater detail with reference to examples. However, the invention is not limited to these examples. The abbreviations used in the examples have the following meanings.
s: singlet
d: doublet
t: triplet
q: quartet
m: multiplet
br: broad
J: coupling constant
Hz: hertz
CDCl₃: deuterated chloroform
DMSO-d₆: deuterated dimethyl sulfoxide
¹H-NMR: proton nuclear magnetic resonance
IR: infrared absorption spectrum

### Example 1

### Production of N-{4'-{[4-butyl-1-(5-methoxypyrimidin-2-yl)-2-methyl-6-oxo-1,6-dihydropyrimidin-5-yl]methyl}biphenyl-2-ylsulfonyl}prop ionamide

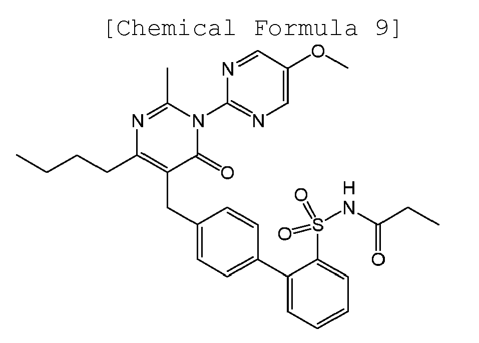

Process 1: The toluene (100 mL)-acetic acid (10.5 mL) solution of methyl 2-(4-bromobenzyl)-3-oxoheptanoate (5.00 g, 15.3 mmol), which has been synthesized according to the method described in EP A 561664, and ammonium acetate (7.08 g, 91.8 mmol) was refluxed for 10 hours under heating. The solvent was distilled off and the resulting residues were added acetic anhydride (10.5 mL) and acetic acid (2.5 mL) under room temperature, and stirred at 70°C for 4 hours. The reaction mixture was added water under ice cooling and extracted with ethyl acetate. The organic layer was combined, washed with water and brine, dried over anhydrous sodium sulfate, and concentrated in vacuo. The residues obtained were subjected to silica gel column chromatography (hexane/ethyl acetate = 3:1), to obtain methyl
(Z)-3-acetamido-2-(4-bromobenzyl)-2-heptenate (2.70 g, 48%) as a pale yellow oil.

¹H-NMR(CDCl₃)δ:
11.82 (1H, s), 7.38 (2H, d, J = 8.5 Hz), 7.00 (2H, d, J = 8.3 Hz), 3.66 (3H, s), 3.63 (2H, s), 2.86 (2H, t, J = 7.9 Hz), 2.16 (3H, s), 1.51-1.42 (2H, m), 1.40-1.30 (2H, m), 0.88 (3H, t, J = 7.3 Hz).

Process 2: Under argon atmosphere, trimethylaluminum (2 mol/L hexane solution, 1.50 mL, 3.00 mmol) was added to 1,2-dichloroethane (6 mL) solution of
2-amino-5-methoxypyrimidine (375 mg, 3.00 mmol) at room temperature, and stirred at the same temperature for 80 minutes. 1,2-dichloroethane (4 mL) solution of methyl (Z)-3-acetamido-2-(4-bromobenzyl)-2-heptenate (369 mg, 1.00 mmol) was added dropwise thereto at room temperature followd by reflux for 17 hours under heating. An aqueous solution of ammonium chloride and chloroform were added to the reaction mixture, which was then filtered through a pad of celite. The organic layer in the filtrate was separated, and the aqueous layer was extracted with chloroform. The organic layer was combined, washed with water and brine, dried over anhydrous sodium sulfate, and concentrated in vacuo. The residues obtained were subjected to silica gel column chromatography (hexane/ethyl acetate = 2:1) to obtain
5-(4-bromobenzyl)-6-butyl-3-(5-methoxypyrimidin-2-yl)-2-met hylpyrimidin-4(3H)-one (410 mg, 93%) as a yellow oil.

¹H-NMR(CDCl₃)δ:
8.54 (2H, s), 7.37-7.33 (2H, m), 7.14 (2H, d, J = 8.5 Hz), 4.00 (3H, s), 3.85 (2H, s), 2.58 (2H, t, J = 7.9 Hz), 2.14 (3H, s), 1.61-1.52 (2H, m), 1.42-1.33 (2H, m), 0.92 (3H, t, J = 7.3 Hz).

Process 3: Under argon atmosphere, aqueous (5 mL) solution of potassium phosphate (1.02 g, 4.81 mmol) and tetrakistriphenylphosphine palladium (64 mg, 0.0555 mmol) were added to 1,4-dioxane (10 mL) solution of
5-(4-bromobenzyl)-6-butyl-3-(5-methoxypyrimidin-2-yl)-2-met hylpyrimidin-4(3H)-one (410 mg, 0.925 mmol) and 2-(N-tert-butylsulfamoyl)phenylboronic acid (571 mg, 2.22 mmol), and refluxed for 4 hours under heating. The reaction mixture was added water and extracted with ethyl acetate. The organic layer was combined, washed with water and brine, dried over anhydrous sodium sulfate, and concentrated in vacuo. The residues obtained were subjected to silica gel column chromatography (hexane/acetone = 5:1) to obtain N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide (360 mg, 68%) as a pale yellow amorphous.

¹H-NMR(CDCl₃)δ:
8.54 (2H, s), 8.15 (1H, dd, J = 7.9, 1.3 Hz), 7.53 (1H, td, J = 7.6, 1.5 Hz), 7.45 (1H, td, J = 7.7, 1.5 Hz), 7.43-7.35 (4H, m), 7.28 (1H, dd, J = 7.7, 1.3 Hz), 4.01 (3H, s), 3.96 (2H, s), 3.53 (1H, s), 2.64 (2H, t, J = 7.9 Hz), 2.16 (3H, s), 1.67-1.58 (2H, m), 1.46-1.36 (2H, m), 0.95 (3H, t, J = 7.3 Hz), 0.95 (9H, s).

Process 4: Trifluoroacetic acid (3.0 mL) and anisole (0.03 mL) was added to
N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide (360 mg, 0.625 mmol) and stirred at room temperature for 24 hours. The reaction mixture was added water and 2 mol/L sodium hydroxide and extracted with ethyl acetate. The organic layer was combined, washed with water and brine, dried over anhydrous sodium sulfate, and concentrated in vacuo. The residues obtained were subjected to silica gel column chromatography (hexane/acetone = 2:3) to obtain 4'-{[4-butyl-1-(5-methoxypyrimidin-2-yl)-2-methyl-6-oxo-1,6 -dihydropyrimidin-5-yl]methyllbiphenyl-2-sulfonamide (314 mg, 97%) as a pale yellow amorphous.

¹H-NMR(CDCl₃)δ:
8.54 (2H, s), 8.15 (1H, dd, J = 8.0, 1.2 Hz), 7.57 (1H, td, J = 7.5, 1.3 Hz), 7.48 (1H, td, J = 7.7, 1.4 Hz), 7.38 (4H, s), 7.31 (1H, dd, J = 7.6, 1.2 Hz), 4.10 (2H, br s), 4.01 (3H, s), 3.96 (2H, s), 2.66 (2H, t, J = 7.8 Hz), 2.16 (3H, s), 1.66-1.57 (2H, m), 1.46-1.36 (2H, m), 0 .94 (3H, t, J = 7.3 Hz).

Process 5: Under argon atmosphere, dichloromethane (0.4 mL) solution of propionyl chloride (8 mg, 0.0866 mmol) was added to dichloromethane (0.6 mL) solution of 4'-{[4-butyl-1-(5-methoxypyrimidin-2-yl)-2-methyl-6-oxo-1,6 -dihydropyrimidin-5-yl]methyl}biphenyl-2-sulfonamide (30 mg, 0.0577 mmol) and triethylamine (0.2 mL) and stirred at room temperature for 15 hours. The reaction mixture was added water and extracted with chloroform. The organic layer was combined, washed with water and brine, dried over anhydrous sodium sulfate, and concentrated in vacuo. The residues obtained were subjected to silica gel column chromatography (chloroform/methanol = 20:1) to obtain N-{4'-{[4-butyl-1-(5-methoxypyrimidin-2-yl)-2-methyl-6-oxo-1,6-dihydropyrimidin-5-yl]methyl}biphenyl-2-ylsulfonyl}prop ionamide (19 mg, 86%) as a colorless oil.

¹H-NMR(CDCl₃)δ:
8.52 (2H, s), 8.26 (1H, dd, J = 8.0, 1.4 Hz), 7.93 (1H, br s), 7.60 (1H, td, J = 8.0, 1.4 Hz), 7.53 (1H, td, J = 7.7, 1.4 Hz), 7.32-7.24 (5H, m), 4.01 (3H, s), 3.95 (2H, s),
2.71 (2H, t, J = 7.8 Hz), 2.16 (3H, s), 1.81 (2H, q, J = 7.4 Hz), 1.71-1.63 (2H, m), 1.49-1.39 (2H, m), 0.96 (3H, t, J = 7.3 Hz), 0.86 (3H, t, J = 7.4 Hz).

### Example 2

### Production of N-{4'-{[4-butyl-1-(5-methoxypyrimidin-2-yl)-2-methyl-6-oxo-1,6-dihydropyrimidin-5-yl]methyl}biphenyl-2-ylsulfonyl}hexa namide

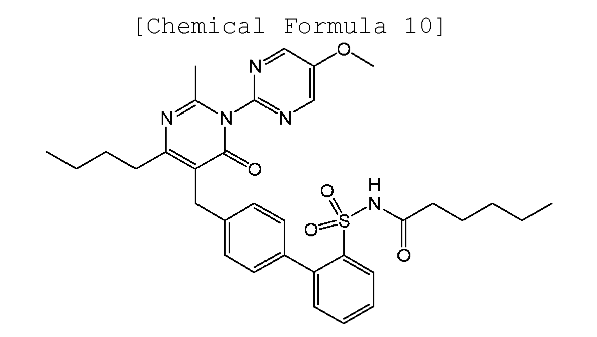

N-{4'-{[4-butyl-1-(5-methoxypyrimidin-2-yl)-2-methyl-6-oxo-1,6-dihydropyrimidin-5-yl]methyl}biphenyl-2-ylsulfony l}hexanamide (yield: 78%) was obtained as a colorless oil according to the same reaction and treatment as the Process 5 of Example 1 by using hexanoyl chloride instead of the propionyl chloride in the Process 5 of Example 1.

¹H-NMR(CDCl₃)δ:
8.53 (2H, s), 8.25 (1H, d, J = 8.0 Hz), 7.63-7.50 (3H, m), 7.32-7.23 (5H, m), 4.01 (3H, s), 3.95 (2H, s), 2.70 (2H, t, J = 7.8 Hz), 2.16 (3H, s), 1.80 (2H, t, J = 7.3 Hz), 1.70-1.62 (2H, m), 1.48-1.39 (2H, m), 1.38-1.30 (2H, m), 1.22-1.15 (2H, m), 1.13-1.07 (2H, m), 0.96 (3H, t, J = 7.3 Hz), 0.82 (3H, t, J = 7.3 Hz).

### Example 3

### Production of N-{4'-{[4-butyl-1-(5-methoxypyrimidin-2-yl)-2-methyl-6-oxo-1,6-dihydropyrimidin-5-yl]methyl}biphenyl-2-ylsulfonyl}cycl opropanecarboxamide

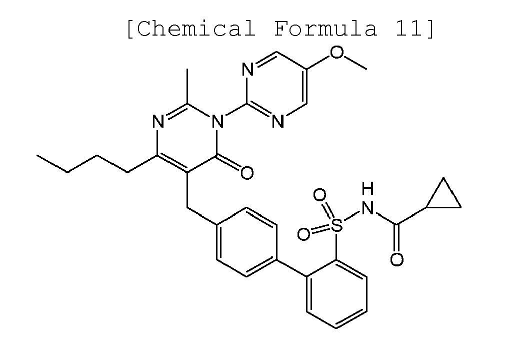

N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopropanecarboxamide (yield: 88%) was obtained as a colorless oil according to the same reaction and treatment as the Process 5 of Example 1 by using cyclopropanecarbonyl chloride instead of the propionyl chloride in the Process 5 of Example 1.

¹H-NMR(CDCl₃)δ:
8.51 (2H, s), 8.23 (1H, dd, J = 8.0, 1.4 Hz), 7.89 (1H, s), 7.60 (1H, td, J = 8.0, 1.4 Hz), 7.51 (1H, td, J = 7.7, 1.4 Hz), 7.31-7.25 (5H, m), 4.01 (3H, s), 3.95 (2H, s), 2.72 (2H, t, J = 7.9 Hz), 2.15 (3H, s), 1.72-1.63 (2H, m), 1.49-1.39 (2H, m), 1.04-0.94 (1H, m), 0.97 (3H, t, J = 7.3 Hz), 0.85-0.80 (2H, m), 0.68-0.63 (2H, m).

### Example 4

### Production of N-{4'-{[4-butyl-1-(5-methoxypyrimidin-2-yl)-2-methyl-6-oxo-1,6-dihydropyrimidin-5-yl]methyl}biphenyl-2-ylsulfonyl}cycl opentanecarboxamide

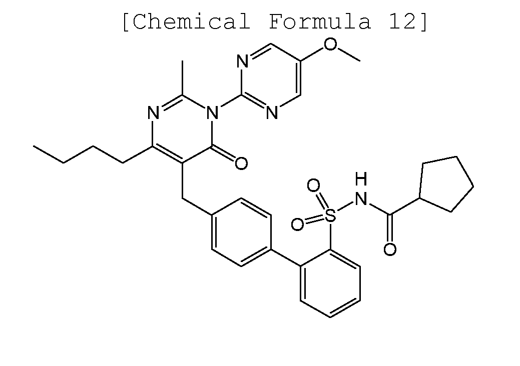

N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopentanecarboxamide (yield: 61%) was obtained as a colorless oil according to the same reaction and treatment as the Process 5 of Example 1 by using cyclopentanecarbonyl chloride instead of the propionyl chloride in the Process 5 of Example 1.

¹H-NMR(CDCl₃)δ:
8.53 (2H, s), 8.26 (1H, dd, J = 8.0, 1.2 Hz), 7.61 (1H, td, J = 7.6, 1.5 Hz), 7.53 (1H, td, J = 7.7, 1.4 Hz), 7.52 (1H, br s), 7.33-7.24 (5H, m), 4.01 (3H, s), 3.95 (2H, s), 2.70 (2H, t, J = 7.9 Hz), 2.16 (3H, s), 2.14-2.07 (1H, m), 1.71-1.63 (2H, m), 1.59-1.37 (10H, m), 0.96 (3H, t, J = 7.3 Hz).

### Example 5

### Production of

### N-{4'-{[4-butyl-1-(5-methoxypyrimidin-2-yl)-2-methyl-6-oxo-1,6-dihydropyrimidin-5-yl]methyl}biphenyl-2-ylsulfonyl}cycl ohexanecarboxamide

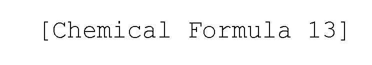

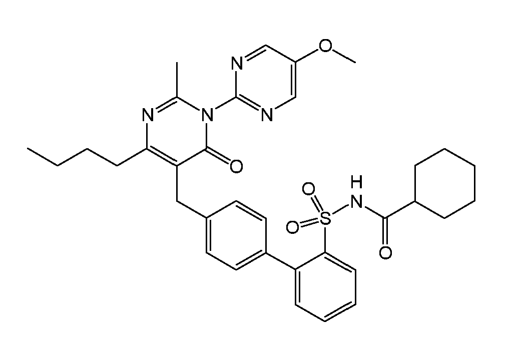

N-{4'-{[4-butyl-1-(5-methoxypyrimidin-2-yl)-2-methyl-6-oxo-1,6-dihydropyrimidin-5-yl]methyl}biphenyl-2-ylsulfony l}cyclohexanecarboxamide (yield: 42%) was obtained as a colorless oil according to the same reaction and treatment as the Process 5 of Example 1 by using cyclohexanecarbonyl chloride instead of the propionyl chloride in the Process 5 of Example 1.

¹H-NMR(CDCl₃)δ:
8.53 (2H, s), 8.27 (1H, dd, J = 7.9, 1.4 Hz), 7.60 (1H, td, J = 7.9, 1.4 Hz), 7.56 (1H, br s), 7.53 (1H, td, J = 7.9, 1.4 Hz), 7.29 (5H, ddd, J = 17.9, 6.7, 3.8 Hz), 4.01 (3H, s), 3.96 (2H, s), 2.70 (2H, t, J = 7.9 Hz), 2.17 (3H, s), 1.76-1.40 (9H, m), 1.21-1.03 (6H, m), 0.97 (3H, t, J = 7.3 Hz).

### Example 6

### Production of N-{4'-{[4-butyl-1-(5-ethoxypyrimidin-2-yl)-2-methyl-6-oxo-1 ,6-dihydropyrimidin-5-yl]methyl}biphenyl-2-ylsulfonyl}propi onamide

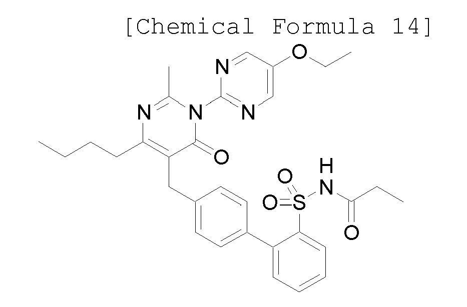

### Process 1:

5-(4-bromobenzyl)-6-butyl-3-(5-ethoxypyrimidin-2-yl)-2-methylpyrimidin-4(3H)-one (yield: 91%) was obtained as a yellow oil according to the same reaction and treatment as the Process 2 of Example 1 by using 2-amino-5-ethoxypyrimidine instead of the 2-amino-5-methoxypyrimidine in the Process 2 of Example 1.

¹H-NMR(CDCl₃)δ:
8.51 (2H, s), 7.37-7.33 (2H, m), 7.16-7.12 (2H, m), 4.22 (2H, q, J = 7.0 Hz), 3.85 (2H, s), 2.60-2.55 (2H, m), 2.14 (3H, s), 1.59-1.52 (2H, m), 1.51 (3H, t, J = 7.0 Hz), 1.42-1.32 (2H, m), 0.91 (3H, t, J = 7.3 Hz).

### Process 2:

N-tert-butyl-4'-{[4-butyl-1-(5-ethoxypyrimidin-2-yl)-2-methyl-6-oxo-1,6-dihydropyrimidin-5-yl]methyl}biphenyl-2-sulfonamide (yield: 58%) was obtained as a colorless amorphous according to the same reaction and treatment as the Process 3 of Example 1 by using 5-(4-bromobenzyl)-6-butyl-3-(5-ethoxypyrimidin-2-yl)-2-meth ylpyrimidin-4 (3H) -one obtained in the Process 1 instead of the 5-(4-bromobenzyl)-6-butyl-3-(5-methoxypyrimidin-2-yl)-2-met hylpyrimidin-4(3H)-one in the Process 3 of Example 1.

¹H-NMR (CDCl₃)**δ**:
8.51 (2H, s), 8.15 (1H, dd, J = 7.9, 1.3 Hz), 7.53 (1H, td, J = 7.4, 1.4 Hz), 7.45 (1H, td, J = 7.7, 1.4 Hz), 7.42-7.35 (4H, m), 7.28 (1H, dd, J = 7.8, 1.5 Hz), 4.22 (2H, q, J = 6.9 Hz), 3.96 (2H, s), 3.53 (1H, s), 2.64 (2H, t, J = 7.9 Hz), 2.16 (3H, s), 1.67-1.58 (2H, m), 1.51 (3H, t, J = 6.9 Hz), 1.46-1.36 (2H, m), 0.95 (3H, t, J = 7.3 Hz), 0.95 (9H, s).

### Process 3:

4'-{[4-butyl-1-(5-ethoxypyrimidin-2-yl)-2-methyl-6-ox o-1,6-dihydropyrimidin-5-yl]methyllbiphenyl-2-sulfonamide (yield: 91%) was obtained as a pale yellow amorphous according to the same reaction and treatment as the Process 4 of Example 1 by using the N-tert-butyl-4'-{[4-butyl-1-(5-ethoxypyrimidin-2-yl)-2-meth yl-6-oxo-1,6-dihydropyrimidin-5-yl]methyl}biphenyl-2-sulfon amide obtained in the Process 2 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR (CDCl₃)**δ**:
8.52 (2H, s), 8.15 (1H, dd, J = 8.0, 1.5 Hz), 7.57 (1H, td, J = 7.5, 1.3 Hz), 7.48 (1H, td, J = 7.7, 1.2 Hz), 7.37 (4H, s), 7.31 (1H, dd, J = 7.4, 1.3 Hz), 4.22 (2H, q, J = 6.9 Hz), 4.10 (2H, s), 3.96 (2H, s), 2.66 (2H, t, J = 7.9 Hz), 2.16 (3H, s), 1.66-1.57 (2H, m), 1.51 (3H, t, J = 7.0 Hz), 1.45-1.36 (2H, m), 0.94 (3H, t, J = 7.3 Hz).

### Process 4:

N-{4'-{[4-butyl-1-(5-ethoxypyrimidin-2-yl)-2-methyl-6 -oxo-1,6-dihydropyrimidin-5-yl]methyl}biphenyl-2-ylsulfonyl }propionamide (yield: 82%) was obtained as a colorless oil according to the same reaction and treatment as the Process 5 of Example 1 by using the 4'-{[4-butyl-1-(5-ethoxypyrimidin-2-yl)-2-methyl-6-oxo-1,6-dihydropyrimidin-5-yl]methyl}biphenyl-2-sulfonamide obtained in the Process 3 instead of the 4'-{[4-butyl-1-(5-methoxypyrimidin-2-yl)-2-methyl-6-oxo-1,6 -dihydropyrimidin-5-yl]methyl}biphenyl-2-sulfonamide in the Process 5 of Example 1.

¹H-NMR(CDCl₃)**δ**:
8.50 (2H, s), 8.26 (1H, dd, J = 8.0, 1.4 Hz), 7.92 (1H, s), 7.60 (1H, td, J = 7.5, 1.4 Hz), 7.52 (1H, td, J = 7.7, 1.4 Hz), 7.32-7.24 (5H, m), 4.22 (2H, q, J = 7.0 Hz), 3.95 (2H, s), 2.70 (2H, t, J = 7.8 Hz), 2.16 (3H, s), 1.81 (2H, q, J = 7.5 Hz), 1.71-1.62 (2H, m), 1.51 (3H, t, J = 7.0 Hz), 1.49-1.39 (2H, m), 0.96 (3H, t, J = 7.3 Hz), 0.85 (3H, t, J = 7.4 Hz).

### Example 7

### Production of N-{4'-{[4-butyl-1-(5-ethoxypyrimidin-2-yl)-2-methyl-6-oxo-1 ,6-dihydropyrimidin-5-yl]methyl}biphenyl-2-ylsulfonyl}hexan amide

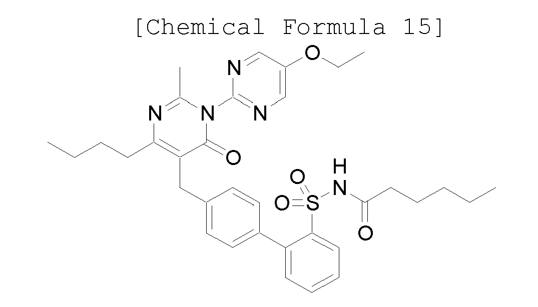

N-{4'-{[4-butyl-1-(5-ethoxypyrimidin-2-yl)-2-methyl-6 -oxo-1,6-dihydropyrimidin-5-yl]methyl}biphenyl-2-ylsulfonyl }hexanamide (yield: 80%) was obtained as a colorless oil according to the same reaction and treatment as the Process 4 of Example 6 by using the hexanoyl chloride instead of the propionyl chloride in the Process 4 of Example 6.

¹H-NMR(CDCl₃)**δ**:
8.50 (2H, s), 8.26 (1H, dd, J = 8.0, 1.3 Hz), 7.82 (1H, s), 7.60 (1H, td, J = 7.5, 1.3 Hz), 7.52 (1H, td, J = 7.7, 1.3 Hz), 7.32-7.23 (5H, m), 4.22 (2H, q, J = 7.0 Hz), 3.95 (2H, s), 2.71 (2H, t, J = 7.9 Hz), 2.16 (3H, s), 1.80 (2H, t, J = 7.4 Hz), 1.72-1.62 (2H, m), 1.51 (3H, t, J = 7.0 Hz), 1.48-1.40 (2H, m), 1.39-1.30 (2H, m), 1.23-1.14 (2H, m), 1.14-1.07 (2H, m), 0.96 (3H, t, J = 7.4 Hz), 0.82 (3H, t, J = 7.2 Hz).

### Example 8

### Production of N-{4'-{[4-butyl-1-(5-ethoxypyrimidin-2-yl)-2-methyl-6-oxo-1 ,6-dihydropyrimidin-5-yl]methyl}biphenyl-2-ylsulfonyl}cyclo propanecarboxamide

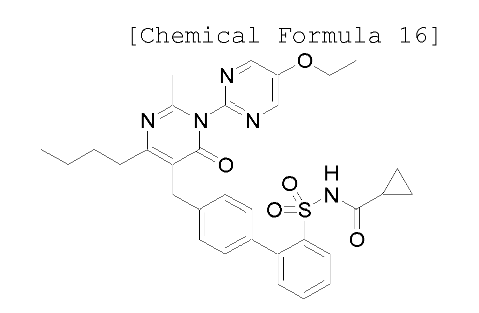

N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopropanecarboxamide (yield: 43%) was obtained as a colorless oil according to the same reaction and treatment as the Process 4 of Example 6 by using cyclopropanecarbonyl chloride instead of the propionyl chloride in the Process 4 of Example 6.

¹H-NMR(CDCl₃)**δ**:
8.48 (2H, s), 8.23 (1H, dd, J = 7.9, 1.3 Hz), 8.02 (1H, s), 7.60 (1H, td, J = 7.6, 1.3 Hz), 7.51 (1H, td, J = 7.6, 1.3 Hz), 7.32-7.25 (5H, m), 4.22 (2H, q, J = 7.0 Hz), 3.95 (2H, s), 2.73 (2H, t, J = 7.9 Hz), 2.16 (3H, s), 1.74-1.65 (2H, m), 1.51 (3H, t, J = 7.1 Hz), 1.49-1.40 (2H, m), 1.04-0.94 (1H, m), 0.97 (3H, t, J = 7.3 Hz), 0.85-0.81 (2H, m), 0.69-0.63 (2H, m).

### Example 9

### Production of N-{4'-{[4-butyl-1-(5-ethoxypyrimidin-2-yl)-2-methyl-6-oxo-1 ,6-dihydropyrimidin-5-yl]methyl}biphenyl-2-ylsulfonyl}cyclo pentanecarboxamide

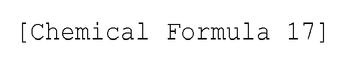

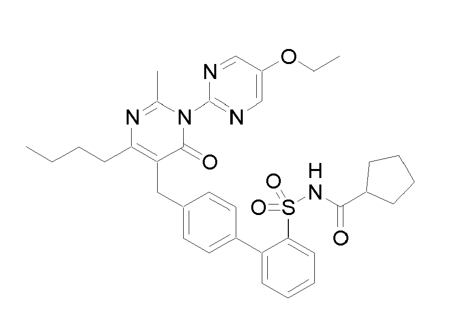

N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopentanecarboxamide (yield: 55%) was obtained as a colorless oil according to the same reaction and treatment as the Process 4 of Example 6 by using cyclopentanecarbonyl chloride instead of the propionyl chloride in the Process 4 of Example 6.

¹H-NMR(CDCl₃)**δ**:
8.50 (2H, s), 8.27 (1H, d, J = 7.8 Hz), 7.77 (1H, s), 7.60 (1H, t, J = 7.3 Hz), 7.53 (1H, t, J = 7.7 Hz), 7.33-7.24 (5H, m), 4.22 (2H, q, J = 7.0 Hz), 3.95 (2H, s), 2.71 (2H, t, J = 7.8 Hz), 2.16 (3H, s), 2.16-2.08 (1H, m), 1.72-1.63 (2H, m), 1.60-1.37 (10H, m), 1.51 (3H, t, J = 7.0 Hz), 0.97 (3H, t, J = 7.2 Hz).

### Example 10

### Production of N-{4'-{[4-butyl-1-(5-ethoxypyrimidin-2-yl)-2-methyl-6-oxo-1 ,6-dihydropyrimidin-5-yl]methyl}biphenyl-2-ylsulfonyl}cyclo hexanecarboxamide

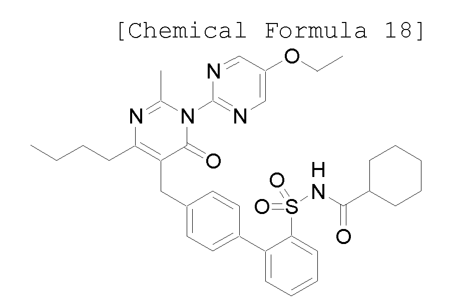

N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclohexanecarboxamide (yield: 47%) was obtained as a colorless oil according to the same reaction and treatment as the Process 4 of Example 6 by using cyclohexanecarbonyl chloride instead of the propionyl chloride in the Process 4 of Example 6.

¹H-NMR(CDCl₃)**δ**:
8.51 (2H, s), 8.27 (1H, dd, J = 8.0, 1.2 Hz), 7.69 (1H, s), 7.60 (1H, td, J = 7.5, 1.3 Hz), 7.52 (1H, td, J = 7.7, 1.3 Hz), 7.33-7.24 (5H, m), 4.22 (2H, q, J = 7.0 Hz), 3.96 (2H, s), 2.70 (2H, t, J = 7.9 Hz), 2.16 (3H, s), 1.74-1.40 (9H, m), 1.51 (3H, t, J = 7.0 Hz), 1.30-1.03 (6H, m), 0.97 (3H, t, J = 7.3 Hz).

### Example 11

### Production of N-{4'-{{4-butyl-1-[5-(difluoromethoxy)pyrimidin-2-yl]-2-met hyl-6-oxo-1,6-dihydropyrimidin-5-yl}methyl}biphenyl-2-ylsul fonyl}cyclopropanecarboxamide

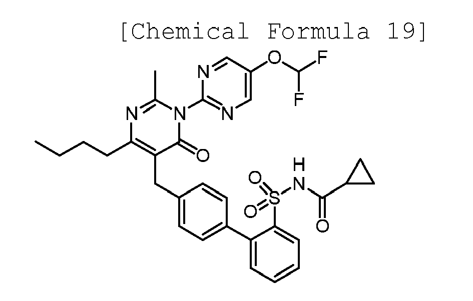

### Process 1:

3-[5-(benzyloxy)pyrimidin-2-yl]-5-(4-bromobenzyl)-6-b utyl-2-methylpyrimidin-4 (3H) -one (yield: 67%) was obtained as a yellow oil according to the same reaction and treatment as the Process 2 of Example 1 by using 2-amino-5-benzyloxypyrimidine instead of the 2-amino-5-methoxypyrimidine in the Process 2 of Example 1.

¹H-NMR(CDCl₃)**δ**:
8.58 (2H, s), 7.45-7.38 (5H, m), 7.35 (2H, d, J = 8.3 Hz), 7.14 (2H, d, J = 8.3 Hz), 5.22 (2H, s), 3.84 (2H, s), 2.58 (2H, t, J = 7.9 Hz), 2.14 (3H, s), 1.61-1.52 (2H, m), 1.44-1.33 (2H, m), 0.91 (3H, t, J = 7.3 Hz).

Process 2: Trifluoroacetic acid (9.0 mL) and anisole (3.0 mL) was added to
3-[5-(benzyloxy)pyrimidin-2-yl]-5-(4-bromobenzyl)-6-butyl-2 -methylpyrimidin-4 (3H) -one (1.05 g, 2.021 mmol) and stirred at 60°C overnight. The reaction mixture was added water and an aqueous solution of saturated sodium hydrocarbonate and extracted with ethyl acetate. The organic layer was combined, washed with water and brine, dried over anhydrous sodium sulfate, and concentrated in vacuo. The residues obtained were subjected to silica gel column chromatography (chloroform/methanol = 10:1) to obtain 5-(4-bromobenzyl)-6-butyl-3-(5-hydroxypyrimidin-2-yl)-2-met hylpyrimidin-4(3H)-one (735 mg, 85%) as a pale yellow solid.

¹H-NMR(CDCl₃)**δ**:
8.14 (2H, s), 7.39 (2H, d, J = 8.3 Hz), 7.10 (2H, d, J = 8.3 Hz), 3.90 (2H, s), 2.61 (2H, t, J = 7.9 Hz), 2.17 (3H, s), 1.62-1.52 (2H, m), 1.43-1.33 (2H, m), 0.91 (3H, t, J=7.3 Hz).

Process 3: Under argon atmosphere, sodium chlorodifluoroacetate (14 mg, 0.09 mmol) and sodium hydroxide (1.3 mg, 0.033 mmol) was added to DMF (1 mL) solution of 5-(4-bromobenzyl)-6-butyl-3-(5-hydroxypyrimidin-2-yl)-2-met hylpyrimidin-4(3H)-one (13 mg, 0.03 mmol) at room temperature and stirred at 55°C overnight. The reaction mixture was added water and extracted with ethyl acetate. The organic layer was combined, washed with water and brine, dried over anhydrous sodium sulfate, and concentrated in vacuo. The residues obtained were subjected to silica gel column chromatography (hexane/ethyl acetate = 1:1) to obtain 5-(4-bromobenzyl)-6-butyl-3-[5-(difluoromethoxy)pyrimidin-2 -yl]-2-methylpyrimidin-4(3H)-one (12 mg, 83%) as a pale yellow oil.

¹H-NMR(CDCl₃)**δ**:
8.79 (2H, s), 7.36 (2H, d, J = 8.3 Hz), 7.14 (2H, d, J = 8.3 Hz), 6.69 (1H, t, J = 70.8 Hz), 3.85 (2H, s), 2.60 (2H, t, J = 7.9 Hz), 2.16 (3H, s), 1.64-1.53 (2H, m), 1.44-1.33 (2H, m), 0.92 (3H, t, J=7.3 Hz).

### Process 4:

N-tert-butyl-4'-{{4-butyl-1-[5-(difluoromethoxy)pyrim idin-2-yl]-2-methyl-6-oxo-1,6-dihydropyrimidin-5-yl}methyl} biphenyl-2-sulfonamide (yield: 96%) was obatined as a pale yellow oil according to the same reaction and treatment as the Process 3 of Example 1 by using the
5-(4-bromobenzyl)-6-butyl-3-[5-(difluoromethoxy)pyrimidin-2 -yl]-2-methylpyrimidin-4(3H)-one obtained in the Process 3 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ**:
8.79 (2H, s), 8.15 (1H, d, J = 7.8 Hz), 7.65-7.25 (7H, m), 6.73 (1H, t, J = 70.8 Hz), 4.93 (1H, brs), 3.97 (2H, s), 2.66 (2H, t, J = 7.9 Hz), 2.18 (3H, s), 1.70-1.59 (2H, m), 1.48-1.36 (2H, m), 0.96-0.93 (12H, m).

### Process 5:

4'-{{4-butyl-1-[5-(difluoromethoxy)pyrimidin-2-yl]-2-methyl-6-oxo-1,6-dihydropyrimidin-5-yl}methyl}biphenyl-2-su lfonamide (yield: 83%) was obtained as a pale yellow oil according to the same reaction and treatment as the Process 4 of Example 1 by using the
N-tert-butyl-4'-{{4-butyl-1-[5-(difluoromethoxy)pyrimidin-2 -yl]-2-methyl-6-oxo-1,6-dihydropyrimidin-5-yl}methyl}biphen yl-2-sulfonamide obtained in the Process 4 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ**:
8.77 (2H, s), 8.09 (1H, d, J = 7.8 Hz), 7.60-7.42 (3H, m), 7.38-7.28 (4H, m), 6.72 (1H, t, J = 71.1 Hz), 4.38-4.30 (2H, br), 3.94 (2H, s), 2.67 (2H, t, J = 7.9 Hz), 2.17 (3H, s), 1.66-1.57 (2H, m), 1.47-1.35 (2H, m), 0.94 (3H, t, J = 7.3 Hz).

### Process 6:

N-{4'-{{4-butyl-1-[5-(difluoromethoxy)pyrimidin-2-yl] -2-methyl-6-oxo-1,6-dihydropyrimidin-5-yl}methyl}biphenyl-2 -ylsulfonyl}cyclopropanecarboxamide (yield: 47%) was obtained as a pale yellow amorphous reaction and treatment as the Process 5 of Example 1 by using the
4'-{{4-butyl-1-[5-(difluoromethoxy)pyrimidin-2-yl]-2-methyl -6-oxo-1,6-dihydropyrimidin-5-yl}methyl}biphenyl-2-sulfonam ide obtained in the Process 5 instead of the
4'-{[4-butyl-1-(5-methoxypyrimidin-2-yl)-2-methyl-6-oxo-1,6 -dihydropyrimidin-5-yl]methyllbiphenyl-2-sulfonamide in the Process 5 of Example 1 and cyclopropanecarbonyl chloride instead of the the propionyl chloride.

¹H-NMR(CDCl₃)**δ**:
8.76 (2H, s), 8.23 (1H, dd, J = 7.8, 0.9 Hz), 7.59 (1H, td, J = 7.6, 1.2 Hz), 7.51 (1H, td, J = 7.6, 1.2 Hz), 7.30-7.24 (5H, m), 6.73 (1H, t, J = 71.0 Hz), 3.95 (2H, s), 2.73 (2H, t, J = 7.9 Hz), 2.17 (3H, s), 1.74-1.64 (2H, m), 1.49-1.40 (2H, m),1.07-0.98 (1H, m), 0.97 (3H, t, J = 7.3 Hz), 0.86-0.80 (2H, m), 0.68-0.62 (2H, m).

### Example 12

### Production of N-{4'-{{4-butyl-2-methyl-6-oxo-1-[5-(2,2,2-trifluoroethoxy) pyrimidin-2-yl]-1,6-dihydropyrimidin-5-yl}methyl}biphenyl-2 -ylsulfonyl}cyclopropanecarboxamide

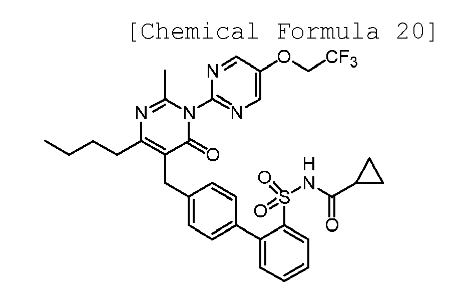

Process 1: Potassium carbonate (332 mg, 2.4 mmol) and 1,1,1-trifluoro-2-iodoethane (252 mg, 1.2 mmol) was added to DMF (3 mL)solution of
5-(4-bromobenzyl)-6-butyl-3-(5-hydroxypyrimidin-2-yl)-2-met hylpyrimidin-4(3H)-one (172 mg, 0.4 mmol) prepared in the Process 2 of Example 11 at room temperature and stirred at 60°C overnight. The reaction mixture was added water and extracted with ethyl acetate. The organic layer was combined, washed with water and brine, dried over anhydrous sodium sulfate, and concentrated in vacuo. The residues obtained were subjected to silica gel column chromatography (hexane/ethyl acetate = 1:1) to obtain
5-(4-bromobenzyl)-6-butyl-2-methyl-3-[5-(2,2,2-trifuluoroet hoxy)pyrimidin-2-yl]pyrimidin-4(3H)-one (77 mg, 38%) as a pale yellow oil.

¹H-NMR(CDCl₃)**δ**:
8.62 (2H, s), 7.36 (2H, d, J = 8.4 Hz), 7.13 (2H, d, J = 8.4 Hz), 4.53 (2H, q, J = 7.6 Hz), 3.85 (2H, s), 2.59 (2H, t, J = 7.9 Hz), 2.15 (3H, s), 1.61-1.53 (2H, m), 1.44-1.33 (2H, m), 0.92 (3H, t, J=7.3 Hz).

### Process 2:

N-tert-butyl-4'-{{4-butyl-2-methyl-6-oxo-1-[5-(2,2,2-trifluoroethoxy)pyrimidin-2-yl]-1,6-dihydropyrimidin-5-yl}m ethyl}biphenyl-2-sulfonamide (yield: 75%) was obtained as a pale yellow oil according to the same reaction and treatment as the Process 3 of Example 1 by using the 5-(4-bromobenzyl)-6-butyl-2-methyl-3-[5-(2,2,2-trifluoroeth oxy)pyrimidin-2-yl]pyrimidin-4(3H)-one obtained in the Process 1 instead of the 5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ**:
8.63 (2H, s), 8.14 (1H, d, J = 7.8 Hz), 7.58-7.25 (7H, m), 4.56 (2H, q, J = 7.7 Hz), 3.97 (2H, s), 3.57 (1H, brs), 2.65 (2H, t, J = 7.9 Hz), 2.16 (3H, s), 1.69-1.59 (2H, m), 1.48-1.36 (2H, m), 0.98-0.93 (12H, m).

### Process 3:

4'-{{4-butyl-2-methyl-6-oxo-1-[5-(2,2,2-trifluoroetho xy)pyrimidin-2-yl]-1,6-dihydropyrimidin-5-yl}methyl}bipheny 1-2-sulfonamide (yield: 93%) was obtained as a pale yellow oil according to the same reaction and treatment as the Process 4 of Example 1 by using the
N-tert-butyl-4'-{{4-butyl-2-methyl-6-oxo-1-[5-(2,2,2-triflu oroethoxy)pyrimidin-2-yl]-1,6-dihydropyrimidin-5-yl}methyl} biphenyl-2-sulfonamide obtained in the Process 2 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ**:
8.60 (2H, s), 8.07 (1H, d, J = 8.0 Hz), 7.58-7.22 (7H, m), 4.55 (2H, q, J = 7.8 Hz), 4.37 (2H, brs), 3.93 (2H, s), 2.66 (2H, t, J = 7.9 Hz), 2.14 (3H, s), 1.67-1.55 (2H, m), 1.46-1.33 (2H, m), 0.93 (3H, t, J = 7.3 Hz).

### Process 4:

N-{4'-{{4-butyl-2-methyl-6-oxo-1-[5-(2,2,2-trifluoroe thoxy)pyrimidin-2-yl]-1,6-dihydropyrimidin-5-yl}methyl}biph enyl-2-ylsulfonyl}cyclopropanecarboxamide (yield: 62%) was obtained as a pale yellow amorphous according to the same reaction and treatment as the Process 5 of Example 1 by using the
4'-{{4-butyl-2-methyl-6-oxo-1-[5-(2,2,2-trifluoroethoxy)pyr imidin-2-yl]-1,6-dihydropyrimidin-5-yl}methyl}biphenyl-2-su lfonamide obtained in the Process 3 instead of the 4'-{[4-butyl-1-(5-methoxypyrimidin-2-yl)-2-methyl-6-oxo-1,6 -dihydropyrimidin-5-yl]methyllbiphenyl-2-sulfonamide in the Process 5 of Example 1 and cyclopropanecarbonyl chloride instead of the propionyl chloride.

¹H-NMR(CDCl₃)**δ**:
8.59 (2H, s), 8.21 (1H, d, J = 7.8 Hz), 7.57 (1H, td, J = 7.4, 1.2 Hz), 7.49 (1H, td, J = 7.4, 1.2 Hz), 7.28-7.22 (5H, m), 4.57 (2H, q, J = 7.8 Hz), 3.94 (2H, s), 2.73 (2H, t, J = 7.8 Hz), 2.15 (3H, s), 1.73-1.62 (2H, m), 1.49-1.39 (2H, m), 1.07-1.00 (1H, m), 0.96 (3H, t, J=7.3 Hz), 0.85-0.77 (2H, m), 0.66-0.59 (2H, m).

### Example 13

### Production of N-f4'-f[4-butyl-l-(5-ethoxypyrimidin-2-yl)-2-ethyl-6-oxo-1, 6-dihydropyrimidin-5-yl]methyl}biphenyl-2-ylsulfonyl}cyclop ropanecarboxamide

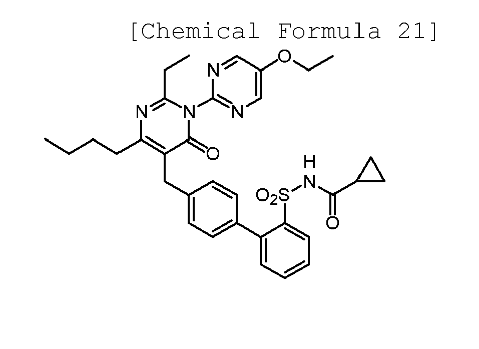

### Process 1: Methyl

(Z)-2-(4-bromobenzyl)-3-propionamido-2-heptenate (yield: 66%) was obtained as a pale yellow oil according to the same reaction and treatment as the Process 1 of Example 1 by using anhydrous propionic acid instead of the anhydrous acetic acid in the Process 1 of Example 1.

¹H-NMR(CDCl₃)**δ**:
11.8 (1H, brs), 7.38 (2H, d, J = 8.5 Hz), 7.01 (2H, d, J = 8.5 Hz), 3.66 (3H, s), 3.63 (2H, s), 2.87 (2H, t, J = 7.9 Hz), 2.54-2.45 (2H, m), 1.52-1.30 (4H, m), 1.24-1.15 (3H, m), 0.88 (3H, t, J=7.2 Hz).

### Process 2:

5-(4-bromobenzyl)-6-butyl-3-(5-ethoxypyrimidin-2-yl)-2-ethylpyrimidin-4 (3H)-one (yield: 66%) was obtained as a pale yellow oil according to the same reaction and treatment as the Process 2 of Example 1 by using methyl
(Z)-2-(4-bromobenzyl)-3-propionamido-2-heptenate instead of the methyl (Z)-3-acetamido-2-(4-bromobenzyl)-2-heptenate and 2-amino-5-ethoxypyrimidine instead of the 2-amino-5-methoxypyrimidine in the Process 2 of Example 1.

¹H-NMR(CDCl₃)**δ**:
8.51 (2H, s), 7.35 (2H, d, J = 8.3 Hz), 7.15 (2H, d, J = 8.3 Hz), 4.22 (2H, q, J = 7.0 Hz), 3.85 (2H, s), 2.60 (2H, t, J = 7.9 Hz), 2.30 (2H, q, J = 7.0 Hz), 1.65-1.54 (2H, m), 1.51 (3H, t, J = 7.0 Hz), 1.42-1.32 (2H, m), 1.16 (3H, t, J = 7.0 Hz), 0.92 (3H, t, J = 7.3 Hz).

### Process 3:

N-tert-butyl-4'-{[4-butyl-1-(5-ethoxypyrimidin-2-yl)-2-ethyl-6-oxo-1,6-dihydropyrimidin-5-yl]methyl}biphenyl-2-s ulfonamide (yield: 82%) was obtained as a pale yellow oil according to the same reaction and treatment as the Process 3 of Example 1 by using the
5-(4-bromobenzyl)-6-butyl-3-(5-ethoxypyrimidin-2-yl)-2-ethy lpyrimidin-4(3H)-one obtained in the Process 2 instead of the 5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ**:
8.51 (2H, s), 8.15 (1H, dd, J = 8.0, 1.0 Hz), 7.57-7.26 (7H, m), 4.22 (2H, q, J = 6.9 Hz), 3.96 (2H, s), 3.57 (1H, brs), 2.66 (2H, t, J = 7.9 Hz), 2.32 (2H, q, J = 7.4 Hz), 1.72-1.60 (2H, m), 1.51 (3H, t, J = 6.9), 1.47-1.35 (2H, m), 1.18 (3H, t, J = 7.4 Hz), 0.99-0.93 (12H, m).

### Process 4:

4'-{[4-butyl-1-(5-ethoxypyrimidin-2-yl)-2-ethyl-6-oxo -1,6-dihydropyrimidin-5-yl]methyl}biphenyl-2-sulfonamide (yield: 76%) was obtained as a pale yellow oil according to the same reaction and treatment as the Process 4 of Example 1 by using the
N-tert-butyl-4'-{[4-butyl-1-(5-ethoxypyrimidin-2-yl)-2-ethy 1-6-oxo-1,6-dihydropyrimidin-5-yl]methyl}biphenyl-2-sulfona mide obtained in the Process 3 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ**:
8.51 (2H, s), 8.12 (1H, dd, J = 7.8, 1.0 Hz), 7.58-7.25 (7H, m), 4.28-4.15 (4H, m), 3.95 (2H, s), 2.68 (2H, t, J = 7.9 Hz), 2.31 (2H, q, J = 7.4 Hz), 1.68-1.59 (2H, m), 1.51 (3H, t, J = 6.9 Hz), 1.46-1.35 (2H, m), 1.17 (3H, t, J = 7.4 Hz), 0.94 (3H, t, J = 7.3 Hz).

### Process 5:

N-{4'-{[4-butyl-1-(5-ethoxypyrimidin-2-yl)-2-ethyl-6-oxo-1,6-dihydropyrimidin-5-yl]methyl}biphenyl-2-ylsulfonyl} cyclopropanecarboxamide (yield: 71%) was obtained as a white amorphous according to the same reaction and treatment as the Process 5 of Example 1 by using the 4'-{[4-butyl-1-(5-ethoxypyrimidin-2-yl)-2-ethyl-6-oxo-1,6-d ihydropyrimidin-5-yllmethyllbiphenyl-2-sulfonamide obtained in the Process 4 instead of the 4'-{[4-butyl-1-(5-methoxypyrimidin-2-yl)-2-methyl-6-oxo-1,6 -dihydropyrimidin-5-yl]methyl}biphenyl-2-sulfonamide in the Process 5 of Example 1 and cyclopropanecarbonyl chloride instead of propionyl chloride.

¹H-NMR(CDCl₃)**δ**:
8.46 (2H, s), 8.22 (1H, d, J = 8.1 Hz), 7.58 (1H, td, J = 7.4, 1.2 Hz), 7.50 (1H, td, J = 7.4, 1.2 Hz), 7.30-7.24 (5H, m), 4.21 (2H, q, J = 6.9 Hz), 3.94 (2H, s), 2.76 (2H, t, J = 7.7 Hz), 2.31 (2H, q, J = 7.3 Hz), 1.78-1.68 (2H, m), 1.45-1.40 (5H, m), 1.18 (3H, t, J=7.3 Hz), 1.15-1.09 (1H, m), 0.97 (3H, t, J = 7.3 Hz), 0.84-0.77 (2H, m), 0.67-0.60 (2H, m).

### Example 14

### Production of N-{4'-{[4-butyl-1-(5-ethoxypyrimidin-2-yl)-2-isopropyl-6-ox o-1,6-dihydropyrimidin-5-yl)methyl)biphenyl-2-ylsulfonyl]cy clopropanecarboxamide

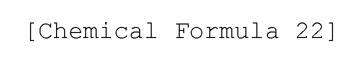

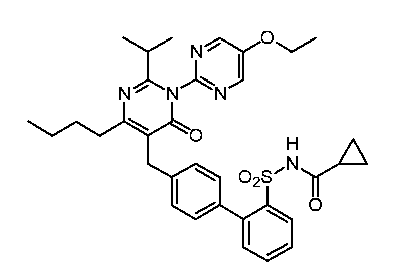

Process 1: The toluene (130 mL)-acetate (16mL) solution of methyl 2-(4-bromobenzyl)-3-oxoheptanoate (8.00 g, 24.4 mmol), which has been synthesized according to the method described in EP A 561664, and ammonium acetate (11.3 g, 146.7 mmol) was refluxed for 6 hours under heating. The solvent was distilled off and a solution of the resulting residue in chloroform (100 mL) was added isobutyryl chloride (3.12 g, 29.3 mmol) and triethylamine (3.20 g, 31.6 mmol) under room temperature, stirred at room temperature for 12 hours, and then stirred at 55°C for 12 hours. The reaction mixture was added water under ice cooling and extracted with ethyl acetate. The organic layer was combined, washed with water and brine, dried over anhydrous sodium sulfate, and concentrated in vacuo. The residues obtained were subjected to silica gel column chromatography (hexane/ethyl acetate = 3:1), to obtain methyl (Z)-2-(4-bromobenzyl)-3-isobutyamido-2-heptenate (3.80 g, 39%) as a yellow oil.

¹H-NMR(CDCl₃)**δ**:
11.9 (1H, brs), 7.38 (2H, d, J = 8.4 Hz), 7.01 (2H, d, J = 8.4 Hz), 3.66 (3H, s), 3.63 (2H, s), 2.87 (2H, t, J = 7.9 Hz), 2.58-2.48 (1H, m), 1.52-1.30 (4H, m), 1.24 (6H, d, J = 6.8 Hz), 0.88 (3H, t, J = 7.2 Hz).

### Process 2:

5-(4-bromobenzyl)-6-butyl-3-(5-ethoxypyrimidin-2-yl)-2-isopropylpyrimidin-4 (3H) -one (yield: 97%) was obtained as a pale yellow oil according to the same reaction and treatment as the Process 2 of Example 1 by using the methyl (Z)-2-(4-bromobenzyl)-3-isobutyramido-2-heptenate obtained in the Process 1 instead of the methyl (Z)-3-acetamido-2-(4-bromobenzyl)-2-heptenate and 2-amino-5-ethoxypyrimidine instead of the 2-amino-5-methoxypyrimidine in the Process 2 of Example 1.

¹H-NMR(CDCl₃)**δ**:
8.50 (2H, s), 7.34 (2H, d, J = 8.3 Hz), 7.16 (2H, d, J = 8.3 Hz), 4.22 (2H, q, J = 7.0 Hz), 3.83 (2H, s), 2.60 (2H, t, J = 7.9 Hz), 2.32-2.22 (1H, m), 1.64-1.56 (2H, m), 1.51 (3H, t, J = 7.0 Hz), 1.41-1.31 (2H, m), 1.18 (6H, d, J = 6.6 Hz), 0.92 (3H, t, J = 7.3 Hz).

### Process 3:

N-tert-butyl-4'-{[4-butyl-1-(5-ethoxypyrimidin-2-yl)-2-isopropyl-6-oxo-1,6-dihydropyrimidin-5-yl]methyl}biphenyl -2-sulfonamide (yield: 72%) was obtained as a pale yellow oil according to the same reaction and treatment as the Process 3 of Example 1 by using the
5-(4-bromobenzyl)-6-butyl-3-(5-ethoxypyrimidin-2-yl)-2-isop ropylpyrimidin-4(3H)-one obtained in the Process 2 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ**:
8.50 (2H, s), 8.15 (1H, d, J = 8.0 Hz), 7.56-7.24 (7H, m), 4.22 (2H, q, J = 6.9 Hz), 3.95 (2H, s), 3.54 (1H, brs), 2.66 (2H, t, J = 7.9 Hz), 2.35-2.24 (1H, m), 1.71-1.61 (2H, m), 1.51 (3H, t, J = 6.9 Hz), 1.46-1.35 (2H, m), 1.20 (6H, d, J = 6.6 Hz), 0.98-0.92 (12H, m).

### Process 4:

4'-{[4-butyl-1-(5-ethoxypyrimidin-2-yl)-2-isopropyl-6 -oxo-1,6-dihydropyrimidin-5-yl]methyl}biphenyl-2-sulfonamid e (yield: 88%) was obtained as a pale yellow oil according to the same reaction and treatment as the Process 4 of Example 1 by using the
N-tert-butyl-4'-{[4-butyl-1-(5-ethoxypyrimidin-2-yl)-2-isop ropyl-6-oxo-1,6-dihydropyrimidin-5-yl]methyl}biphenyl-2-sul fonamide obtained in the Process 3 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ**:
8.51 (2H, s), 8.14 (1H, d, J = 7.8 Hz), 7.59-7.24 (7H, m), 4.22 (2H, q, J = 6.9 Hz), 4.13 (2H, brs), 3.95 (2H, s), 2.68 (2H, t, J = 7.9 Hz), 2.33-2.24 (1H, m), 1.71-1.59 (2H, m), 1.51 (3H, t, J = 6.9 Hz), 1.45-1.34 (2H, m), 1.20 (6H, d, J = 6.8 Hz), 0.94 (3H, t, J = 7.3 Hz).

### Process 5:

N-{4'-{[4-butyl-1-(5-ethoxypyrimidin-2-yl)-2-isopropy 1-6-oxo-1,6-dihydropyrimidin-5-yl)methyl)biphenyl-2-ylsulfo nyl)cyclopropanecarboxamide (yield: 81%) was obtained as a white amorphous according to the same reaction and treatment as the Process 5 of Example 1 by using the 4'-{[4-butyl-1-(5-ethoxypyrimidin-2-yl)-2-isopropyl-6-oxo-1 ,6-dihydropyrimidin-5-yl]methyl}biphenyl-2-sulfonamide obtained in the Process 4 instead of the 4'-{[4-butyl-1-(5-methoxypyrimidin-2-yl)-2-methyl-6-oxo-1,6 -dihydropyrimidin-5-yl]methyl}biphenyl-2-sulfonamide in the Process 5 of Example 1 and cyclopropanecarbonyl chloride instead of the propionyl chloride.

¹H-NMR(CDCl₃)**δ**:
8.46 (2H, s), 8.23 (1H, d, J = 8.0 Hz), 7.59 (1H, td, J = 7.6, 1.2 Hz), 7.51 (1H, td, J = 7.6, 1.2 Hz), 7.32-7.25 (5H, m), 4.21 (2H, q, J = 6.9 Hz), 3.93 (2H, s), 2.77 (2H, t, J = 7.7 Hz), 2.32-2.23 (1H, m), 1.78-1.68 (2H, m), 1.51 (3H, t, J = 6.9 Hz), 1.50-1.40 (2H, m), 1.19 (6H, d, J = 6.6 Hz), 1.02-0.94 (4H, m), 0.84-0.78 (2H, m), 0.66-0.58 (2H, m).

### Example 15

### Production of N-{4'-{[4-butyl--2-cyclopropyl-1-(5-methoxypyrimidin-2-yl)-6-oxo-1,6-dihydropyrimidin-5-yl)methyl)biphenyl-2-ylsulfony 1]cyclopropanecarboxamide

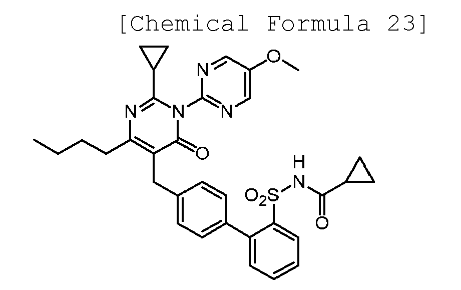

### Process 1: Methyl

(Z)-2-(4-bromobenzyl)-3-(cyclopropanecarboxamide)-2-heptena te (yield: 71%) was obtaind as a pale yellow oil according to the same reaction and treatment as the Process 1 of Example 14 by using cyclopropanecarbonyl chloride instead of the isobutyryl chloride in the Process 1 of Example 14.

¹H-NMR(CDCl₃)**δ**:
12.1 (1H, brs), 7.38 (2H, d, J = 8.4 Hz), 7.13 (2H, d, J = 8.4 Hz), 3.67 (3H, s), 3.63 (2H, s), 2.85 (2H, t, J = 7.9 Hz), 1.64-1.24 (5H, m), 1.06-0.80 (7H, m).

### Process 2:

5-(4-bromobenzyl)-6-butyl-2-cyclopropyl-3-(5-methoxyp yrimidin-2-yl)pyrimidin-4(3H)-one (yield: 41%) was obtained as a pale yellow oil according to the same reaction and treatment as the Process 2 of Example 1 by using the methyl (Z)-2-(4-bromobenzyl)-3-(cyclopropanecarboxamide)-2-heptena te obtained in the Process 1 instead of the methyl (Z)-3-acetamido-2-(4-bromobenzyl)-2-heptenate in the Process 2 of Example 1.

¹H-NMR(CDCl₃)**δ**:
8.55 (2H, s), 7.33 (2H, d, J = 8.5 Hz), 7.14 (2H, d, J = 8.5 Hz), 3.99 (3H, s), 3.82 (2H, s), 2.52 (2H, t, J = 7.9 Hz), 1.58-1.48 (2H, m), 1.38-1.17 (4H, m), 1.14-1.06 (1H, m), 0.89 (3H, t, J = 7.3 Hz), 0.85-0.78 (2H, m).

### Process 3:

N-tert-butyl-4'-{[4-butyl-2-cyclopropyl-1-(5-methoxyp yrimidin-2-yl)-6-oxo-1,6-dihydropyrimidin-5-yl]methyl}biphe nyl-2-sulfonamide (yield: 55%) was obtained as a pale yellow oil according to the same reaction and treatment as the Process 3 of Example 1 by using the
5-(4-bromobenzyl)-6-butyl-2-cyclopropyl-3-(5-methoxypyrimid in-2-yl)pyrimidin-4 (3H)-one obtained in the Process 2 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ**:
8.56 (2H, s), 8.14 (1H, d, J = 1.2, 7.8 Hz), 7.56-7.26 (7H, m), 4.00 (3H, s), 3.94 (2H, s), 3.56 (1H, brs), 2.58 (2H, t, J = 7.9 Hz), 1.64-1.54 (2H, m), 1.42-1.32 (2H, m), 1.26-1.19 (2H, m), 1.15-1.07 (1H, m), 0.96-0.81 (14H, m).

### Process 4:

4'-{[4-butyl-2-cyclopropyl-1-(5-methoxypyrimidin-2-yl )-6-oxo-1,6-dihydropyrimidin-5-yl]methyl}biphenyl-2-sulfona mide (yield: 83%) was obtained as a pale yellow oil according to the same reaction and treatment as the Process 4 of Example 1 by using the N-tert-butyl-4'-{[4-butyl-2-cyclopropyl-1-(5-methoxypyrimid in-2-yl)-6-oxo-1,6-dihydropyrimidin-5-yl]methyl}biphenyl-2-sulfonamide obtained in the Process 3 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ**:
8.56 (2H, s), 8.14 (1H, d, J = 8.0 Hz),7.59-7.44 (2H, m), 7.38-7.28 (5H, m), 4.11 (2H, brs), 4.01 (3H, s), 3.94 (2H, s), 2.61 (2H, t, J = 7.9 Hz), 1.62-1.53 (2H, m), 1.62-1.53 (2H, m), 1.42-1.21 (2H, m), 1.15-1.07 (1H, m), 0.92 (3H, t, J = 7.3 Hz), 0.87-0.81 (2H, m).

### Process 5:

N-{4'-{[4-butyl--2-cyclopropyl-1-(5-methoxypyrimidin-2-yl)-6-oxo-1,6-dihydropyrimidin-5-yl]methyl}biphenyl-2-yls ulfonyl}cyclopropanecarboxamide (yield: 82%) was obtained as a white amorphous according to the same reaction and treatment as the Process 5 of Example 1 by using the
4'-{[4-butyl-2-cyclopropyl-1-(5-methoxypyrimidin-2-yl)-6-ox o-l,6-dihydropyrimidin-5-yl]methyl}biphenyl-2-sulfonamide obtained in the Process 4 instead of the
4'-{[4-butyl-1-(5-methoxypyrimidin-2-yl)-2-methyl-6-oxo-1,6 -dihydropyrimidin-5-yl]methyl}biphenyl-2-sulfonamide and cyclopropanecarbonyl chloride instead of the propionyl chloride in the Process 5 of Example 1.

¹H-NMR(CDCl₃)**δ:**
8.52 (2H, s), 8.24 (1H, dd, J = 8.1, 1.2 Hz), 7.59 (1H, td, J = 7.4, 1.2 Hz), 7.51 (1H, td, J = 7.4, 1.2 Hz), 7.32-7.23 (5H, m), 4.00 (3H, s), 3.93 (2H, s), 2.69 (2H, t, J = 7.7 Hz), 1.72-1.62 (2H, m), 1.47-1.36 (2H, m), 1.27-1.20 (2H, m), 1.28-1.07 (2H, m), 0.96 (3H, t, J = 7.3 Hz), 0.88-0.80 (4H, m), 0.68-0.63 (2H, m).

### Example 16

### Production of N-{4'-{[4-butyl-2-cyclopropyl-1-(5-ethoxypyrimidin-2-yl)-6-oxo-1,6-dihydropyrimidin-5-yl]methyl}biphenyl-2-ylsulfonyl} cyclopropanecarboxamide

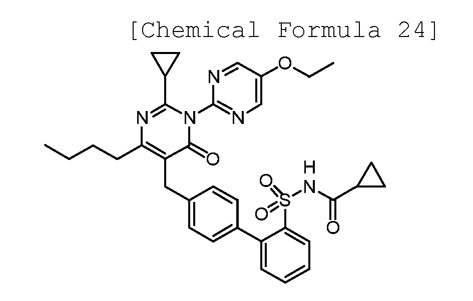

### Process 1:

5-(4-bromobenzyl)-6-butyl-2-cyclopropyl-3-(5-ethoxypy rimidin-2-yl)pyrimidin-4(3H)-one (yield: 38%) was obtained as a yellow oil according to the same reaction and treatment as the Process 2 of Example 15 by using 2-amino-5-ethoxypyrimidine instead of the 2-amino-5-methoxypyrimidine in the Process 2 of Example 15.

¹H-NMR(CDCl₃)**δ**:
8.53 (2H, s), 7.34 (2H, d, J = 8.3 Hz), 7.13 (2H, d, J = 8.3 Hz), 4.21 (2H, q, J = 7.0 Hz), 3.82 (2H, s), 2.52 (2H, t, J = 7.9 Hz), 1.57-1.46 (5H, m), 1.38-1.28 (2H, m), 1.23-1.17 (2H, m), 1.13-1.06 (1H, m), 0.89 (3H, t, J = 7.3 Hz), 0.85-0.77 (2H, m).

### Process 2:

N-tert-butyl-4'-{[4-butyl-2-cyclopropyl-1-(5-ethoxypy rimidin-2-yl)-6-oxo-1,6-dihydropyrimidin-5-yl]methyl}biphen yl-2-sulfonamide (yield: 47%) was obtained as a yellow oil according to the same reaction and treatment as the Process 3 of Example 1 by using the
5-(4-bromobenzyl)-6-butyl-2-cyclopropyl-3-(5-ethoxypyrimidi n-2-yl)pyrimidin-4(3H)-one obtained in the Process 1 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ:**
8.54 (2H, s), 8.13 (1H, d, J = 8.0 Hz), 7.57-7.25 (7H, m), 4.22 (2H, q, J = 6.9 Hz), 3.94 (2H, s), 3.58 (1H, brs), 2.58 (2H, t, J = 7.9 Hz), 1.64-1.55 (2H, m), 1.51 (3H, t, J = 6.9 Hz), 1.42-1.32 (2H, m), 1.26-1.20 (2H, m), 1.16-1.08 (1H, m), 0.98-0.82 (14H, m).

### Process 3:

4'-{[4-butyl-2-cyclopropyl-1-(5-ethoxypyrimidin-2-yl) -6-oxo-1,6-dihydropyrimidin-5-yl]methyl}biphenyl-2-sulfonam ide (yield: 81%) was obtained as a pale yellow oil according to the same reaction and treatment as the Process 4 of Example 1 by using the
N-tert-butyl-4'-{[4-butyl-2-cyclopropyl-1-(5-ethoxypyrimidi n-2-yl)-6-oxo-1,6-dihydropyrimidin-5-yl]methyl}biphenyl-2-s ulfonamide obtained in the Process 2 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ:**
8.52 (2H, s), 8.09 (1H, d, J = 8.1 Hz), 7.57-7.26 (7H, m), 4.34 (2H, brs), 4.21 (2H, q, J = 6.9 Hz), 3.92 (2H, s), 2.60 (2H, t, J = 7.9 Hz), 1.62-1.53 (2H, m), 1.50 (3H, t, J = 6.9 Hz), 1.41-1.31 (2H, m), 1.25-1.18 (2H, m), 1.15-1.08 (1H, m), 0.92 (3H, t, J = 7.3 Hz), 0.87-0.80 (2H, m).

### Process 4:

N-{4'-{[4-butyl-2-cyclopropyl-1-(5-ethoxypyrimidin-2-yl)-6-oxo-1,6-dihydropyrimidin-5-yl]methyl}biphenyl-2-ylsul fonyl}cyclopropanecarboxamide (yield: 71%) was obtained as a pale yellow amorphous according to the same reaction and treatment as the Process 5 of Example 1 by using the 4'-{[4-butyl-2-cyclopropyl-1-(5-ethoxypyrimidin-2-yl)-6-oxo -1,6-dihydropyrimidin-5-yl]methyl}biphenyl-2-sulfonamide obtained in the Process 3 instead of the 4'-{[4-butyl-1-(5-methoxypyrimidin-2-yl)-2-methyl-6-oxo-1,6 -dihydropyrimidin-5-yl]methyl}biphenyl-2-sulfonamide in the Process 5 of Example 1 and cyclopropanecarbonyl chloride instead of the propionyl chloride.

¹H-NMR(CDCl₃)**δ:**
8.49 (2H, s), 8.24 (1H, d, J = 8.0 Hz), 7.59 (1H, td, J = 7.4, 1.2 Hz), 7.51 (1H, td, J = 7.4, 1.2 Hz), 7.31-7.23 (5H, m), 4.21 (2H, q, J = 6.9 Hz), 3.92 (2H, s), 2.69 (2H, t, J = 7.7 Hz), 1.72-1.63 (2H, m), 1.50 (3H, t, J = 6.9 Hz), 1.47-1.35 (2H, m), 1.28-1.20 (2H, m), 1.15-1.08 (2H, m), 0.96 (3H, t, J=7.3 Hz), 0.88-0.79 (4H, m), 0.69-0.62 (2H, m).

### Example 17

### Production of N-{4'-{[4-butyl-2-cyclobutyl-1-(5-ethoxypyrimidin-2-yl)-6-0 xo-1,6-dihydropyrimidin-5-yl]methyl}biphenyl-2-ylsulfonyl}c yclopropanecarboxamide

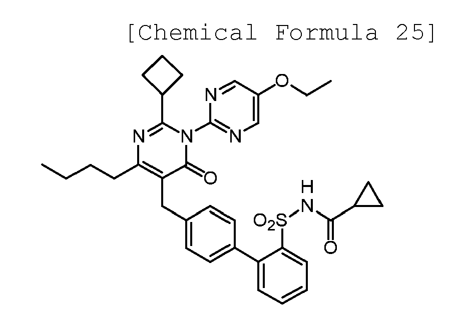

### Process 1: Methyl

(Z)-2-(4-bromobenzyl)-3-(cyclobutanecarboxamide)-2-heptenat e (yield: 48%) was obtained as a pale yellow oil according to the same reaction and treatment as the Process 1 of Example 14 by using cyclobutanecarbonyl chloride instead of the isobutyryl chloride in the Process 1 of Example 14.

¹H-NMR(CDCl₃)**δ:**
11.7 (1H, brs), 7.38 (2H, d, J = 8.4 Hz), 7.01 (2H, d, J = 8.4 Hz), 3.65 (3H, s), 3.63 (2H, s), 3.25-3.06 (1H, m), 2.87 (2H, t, J = 7.9 Hz), 2.44-1.85 (6H, m), 1.53-1.31 (4H, m), 0.88 (3H, t, J=7.2 Hz).

### Process 2:

5-(4-bromobenzyl)-6-butyl-2-cyclobutyl-3-(5-ethoxypyr imidin-2-yl)pyrimidin-4(3H)-one (yield: 99%) was obtaind as a pale yellow oil according to the same reaction and treatment as the Process 2 of Example 1 by using the methyl (Z)-2-(4-bromobenzyl)-3-(cyclobutanecarboxamide)-2-heptenat e obtained in the Process 1 instead of the methyl (Z)-3-acetamido-2-(4-bromobenzyl)-2-heptenate and 2-amino-5-ethoxypyrimidine instead of the 2-amino-5-methoxypyrimidine in the Process 2 of Example 1.

¹H-NMR(CDCl₃)**δ:**
8.49 (2H, s), 7.34 (2H, d, J = 8.3 Hz), 7.15 (2H, d, J = 8.3 Hz), 4.21 (2H, q, J = 7.0 Hz), 3.85 (2H, s), 3.16-3.46 (1H, m), 2.62 (2H, t, J = 7.9 Hz), 2.48-2.36 (2H, m), 1.80-1.56 (6H, m), 1.51 (3H, t, J = 7.0 Hz), 1.44-1.34 (2H, m), 0.93 (3H, t, J = 7.3 Hz).

### Process 3:

N-tert-butyl-4'-{[4-butyl-2-cyclobutyl-1-(5-ethoxypyr imidin-2-yl)-6-oxo-1,6-dihydropyrimidin-5-yl]methyl}bipheny 1-2-sulfonamide (yield: 72%) was obtained as a pale yellow oil according to the same reaction and treatment as the Process 3 of Example 1 by using the
5-(4-bromobenzyl)-6-butyl-2-cyclobutyl-3-(5-ethoxypyrimidin -2-yl)pyrimidin-4(3H)-one obtained in thte Process 2 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ:**
8.49 (2H, s), 8.15 (1H, dd, J = 7.8, 1.2 Hz), 7.56-7.35 (6H, m), 7.29-7.26 (1H, m), 4.22 (2H, q, J = 6.9 Hz), 3.96 (2H, s), 3.56 (1H, s), 3.18-3.06 (1H, m), 2.68 (2H, t, J = 7.9 Hz), 2.52-2.39 (2H, m), 1.82-1.64 (6H, m), 1.51 (3H, t, J = 6.9 Hz), 1.48-1.38 (2H, m), 0.98-0.92 (12H, m).

### Process 4:

4'-{[4-butyl-2-cyclobutyl-1-(5-ethoxypyrimidin-2-yl)-6-oxo-1,6-dihydropyrimidin-5-yl]methyl}biphenyl-2-sulfonami de (yield: 100%) was obtained as a pale yellow oil according to the same reaction and treatment as the Process 4 of Example 1 by using the the
N-tert-butyl-4'-{[4-butyl-2-cyclobutyl-1-(5-ethoxypyrimidin -2-yl)-6-oxo-1,6-dihydropyrimidin-5-yl]methyl}biphenyl-2-su lfonamide obtained in the Process 3 instead of the
N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ:**
8.49 (2H, s), 8.14 (1H, dd, J = 7.9, 1.1 Hz), 7.57 (1H, td, J = 7.4, 1.3 Hz), 7.47 (1H, td, J = 7.4, 1.3 Hz), 7.39-7.29 (5H, m), 4.22 (2H, q, J = 6.9 Hz), 4.15 (2H, brs), 3.96 (2H, s), 3.17-3.06 (1H, m), 2.71 (2H, t, J = 7.9 Hz), 2.52-2.39 (2H, m), 1.82-1.60 (6H, m), 1.51 (3H, t, J=6.9 Hz), 1.47-1.36 (2H, m), 0.95 (3H, t, J = 7.5 Hz).

### Process 5:

N-{4'-{[4-butyl-2-cyclobutyl-1-(5-ethoxypyrimidin-2-y 1)-6-oxo-1,6-dihydropyrimidin-5-yl]methyl}biphenyl-2-ylsulf onyl}cyclopropanecarboxamide (yield: 69%) was obtained as a white amorphous according to the same reaction and treatment as the Process 5 of Example 1 by using the 4'-{[4-butyl-2-cyclobutyl-1-(5-ethoxypyrimidin-2-yl)-6-oxo-1,6-dihydropyrimidin-5-yl]methyl}biphenyl-2-sulfonamide obtained in the Process 4 instead of the 4'-{[4-butyl-1-(5-methoxypyrimidin-2-yl)-2-methyl-6-oxo-1,6 -dihydropyrimidin-5-yl]methyl}biphenyl-2-sulfonamide and cyclopropanecarbonyl chloride instead of the propionyl chloride in the Process 5 of Example 1.

¹H-NMR(CDCl₃)**δ:**
8.45 (2H, s), 8.23 (1H, d, J = 8.0 Hz), 7.59 (1H, td, J = 7.4, 1.2 Hz), 7.51 (1H, td, J = 7.4, 1.2 Hz), 7.33-7.23 (5H, m), 4.21 (2H, q, J = 6.9 Hz), 3.95 (2H, s), 3.15-3.04 (1H, m), 2.79 (2H, t, J = 7.7 Hz), 2.49-2.38 (2H, m), 1.82-1.71 (6H, m), 1.51 (3H, t, J = 6.9 Hz), 1.50-1.42 (2H, m), 1.03-0.95 (4H, m), 0.84-0.78 (2H, m), 0.66-0.59 (2H, m).

### Example 18

### Production of N-{4'-{[4-butyl-2-cyclopentyl-1-(5-ethoxypyrimidin-2-yl)-6-oxo-1,6-dihydropyrimidin-5-yl]methyl}biphenyl-2-ylsulfonyl} cyclopropanecarboxamide

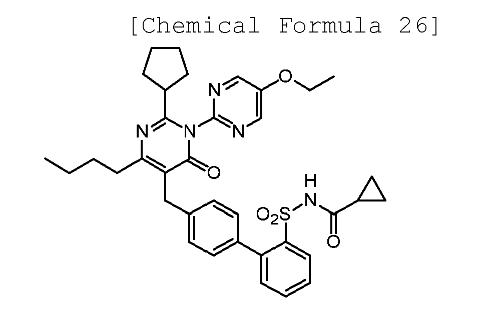

### Process 1: Methyl

(Z)-2-(4-bromobenzyl)-3-(cyclopentanecarboxamide)-2-heptena te (yield: 34%) was obtained as a pale yellow oil acording to the same reaction and treatment as the Process 1 of Example 14 by using cyclopentanecarbonyl chloride instead of the isobutyryl chloride in the Process 1 of Example 14.

¹H-NMR(CDCl₃)**δ:**
11.9 (1H, brs), 7.38 (2H, d, J = 8.5 Hz), 7.01 (2H, d, J = 8.5 Hz), 3.66 (3H, s), 3.63 (2H, s), 2.87 (2H, t, J = 7.9 Hz), 2.78-2.68 (1H, m), 2.04-1.32 (12H, m), 0.87 (3H, t, J = 7.2 Hz).

### Process 2:

5-(4-bromobenzyl)-6-butyl-2-cyclopentyl-3-(5-ethoxypy rimidin-2-yl)pyrimidin-4(3H)-one (yield: 83%)was obtained as a pale yellow oil according to the same reaction and treatment as the Process 2 of Example 1 by using the methyl
(Z)-2-(4-bromobenzyl)-3-(cyclopentanecarboxamide)-2-heptena te obtained in the Process 1 instead of the methyl (Z)-3-acetamido-2-(4-bromobenzyl)-2-heptenate and 2-amino-5-ethoxypyrimidine instead of the
2-amino-5-methoxypyrimidine in the Process 2 of Example 1.

¹H-NMR(CDCl₃)**δ:**
8.50 (2H, s), 7.34 (2H, d, J = 8.3 Hz), 7.15 (2H, d, J = 8.3 Hz), 4.21 (2H, q, J = 7.0 Hz), 3.84 (2H, s), 2.59 (2H, t, J = 7.9 Hz), 2.48-2.39 (1H, m), 2.02-1.91 (2H, m), 1.78-1.22 (13H, m), 0.91 (3H, t, J = 7.3 Hz).

### Process 3:

N-tert-butyl-4'-{[4-butyl-2-cyclopentyl-1-(5-ethoxypy rimidin-2-yl)-6-oxo-1,6-dihydropyrimidin-5-yl]methyl}biphen yl-2-sulfonamide (yield: 60%) was obtained as a pale yellow oil according to the same reaction and treatment as the Process 3 of Example 1 by using the
5-(4-bromobenzyl)-6-butyl-2-cyclopentyl-3-(5-ethoxypyrimidi n-2-yl)pyrimidin-4(3H)-one obtained in the Process 2 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ:**
8.51 (2H, s), 8.14 (1H, dd, J = 7.8, 1.0 Hz),
7.57-7.34 (6H, m), 7.32-7.24 (1H, m), 4.22 (2H, q, J=7.0 Hz), 3.95 (2H, s), 3.58 (1H, brs), 2.65 (2H, t, J = 7.9 Hz), 2.50-2.40 (1H, m), 2.05-1.92 (2H, m), 1.82-1.60 (8H, m), 1.55-1.33 (5H, m), 0.98-0.93 (12H, m).

### Process 4:

4'-{[4-butyl-2-cyclopentyl-1-(5-ethoxypyrimidin-2-yl) -6-oxo-1,6-dihydropyrimidin-5-yl]methyl}biphenyl-2-sulfonam ide (yield: 94%) was obtained as a pale yellow oil according to the same reaction and treatment as the Process 4 of Example 1 by using the
N-tert-butyl-4'-{[4-butyl-2-cyclopentyl-1-(5-ethoxypyrimidi n-2-yl)-6-oxo-1,6-dihydropyrimidin-5-yl]methyl}biphenyl-2-s ulfonamide obtained in the Process 3 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ:**
8.49 (2H, s), 8.10 (1H, dd, J = 7.9, 1.4 Hz), 7.58-7.42 (2H, m), 7.36-7.26 (5H, m), 4.26 (2H, brs), 4.21 (2H, q, J = 7.0 Hz), 3.93 (2H, s), 2.67 (2H, t, J = 7.9 Hz), 2.49-2.39 (1H, m), 2.02-1.93 (2H, m), 1.79-1.62 (6H, m), 1.53-1.33 (7H, m), 0.93 (3H, t, J = 6.9 Hz).

### Process 5:

N-{4'-{[4-butyl-2-cyclopentyl-1-(5-ethoxypyrimidin-2-yl)-6-oxo-1,6-dihydropyrimidin-5-yl]methyl}biphenyl-2-ylsul fonyl}cyclopropanecarboxamide (yield: 69%) was obtained as a white amorphous according to the same reaction and treatment as the Process 5 of Example 1 by using the 4'-{[4-butyl-2-cyclopentyl-1-(5-ethoxypyrimidin-2-yl)-6-oxo -1,6-dihydropyrimidin-5-yl]methyl}biphenyl-2-sulfonamide obtained in the Process 4 instead of the
4'-{[4-butyl-1-(5-methoxypyrimidin-2-yl)-2-methyl-6-oxo-1,6 -dihydropyrimidin-5-yl]methyl}biphenyl-2-sulfonamide and cyclopropanecarbonyl chloride instead of the propionyl chloride in the Process 5 of Example 1.

¹H-NMR(CDCl₃)**δ:**
8.46 (2H, s), 8.23 (1H, d, J = 8.0 Hz), 7.58 (1H, td, J = 7.4, 1.2 Hz), 7.50 (1H, td, J = 7.4, 1.2 Hz), 7.32-7.22 (5H, m), 4.22 (2H, q, J = 6.9 Hz), 3.93 (2H, s), 2.76 (2H, t, J = 7.6 Hz), 2.03-1.91 (1H, m), 2.03-1.91 (2H, m), 1.82-1.63 (6H, m), 1.54-1.38 (7H, m), 1.15-0.94 (4H, m), 0.84-0.78 (2H, m), 0.66-0.59 (2H, m).

### Example 19

### Production of N-{4'-{[4-butyl-2-cyclohexyl-1-(5-ethoxypyrimidin-2-yl)-6-o xo-1,6-dihydropyrimidin-5-yl]methyl}biphenyl-2-ylsulfonyl}c yclopropanecarboxamide

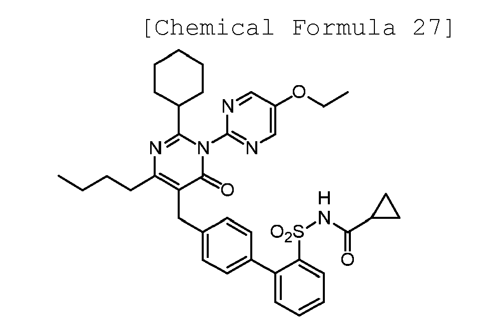

### Process 1: Methyl

(Z)-2-(4-bromobenzyl)-3-(cyclohexanecarboxamide)-2-heptenat e (yield: 36%) was obtained as a pale yellow oil according to the same reaction and treatment as the Process 1 of Example 14 by using cyclohexanecarbonyl chloride instead of the isobutyryl chloride in the Process 1 of Example 14.

¹H-NMR(CDCl₃)**δ:**
11.8 (1H, brs), 7.38 (2H, d, J = 8.5 Hz), 7.01 (2H, d, J = 8.5 Hz), 3.66 (3H, s), 3.63 (2H, s), 2.87 (2H, t, J = 7.9 Hz), 2.32-2.21 (1H, m), 2.01-1.22 (12H, m), 0.92-0.85 (5H, m).

### Process 2:

5-(4-bromobenzyl)-6-butyl-2-cyclohexyl-3-(5-ethoxypyr imidin-2-yl)pyrimidin-4(3H)-one (yield: 100%) was obtained as a pale yellow oil according to the same reaction and treatment as the Process 2 of Example 1 by using the methyl (Z)-2-(4-bromobenzyl)-3-(cyclohexanecarboxamide)-2-heptenat e obtained in the Process 1 instead of the methyl (Z)-3-acetamido-2-(4-bromobenzyl)-2-heptenate and 2-amino-5-ethoxypyrimidine instead of the
2-amino-5-methoxypyrimidine in the Process 2 of Example 1.

¹H-NMR(CDCl₃)**δ:**
8.51 (2H, s), 7.34 (2H, d, J = 8.5 Hz), 7.15 (2H, d, J = 8.5 Hz), 4.22 (2H, q, J = 7.0 Hz), 3.83 (2H, s), 2.59 (2H, t, J = 7.9 Hz), 1.90-1.15 (16H, m), 1.04-0.88 (5H, m).

### Process 3:

N-tert-butyl-4'-{[4-butyl-2-cyclohexyl-1-(5-ethoxypyr imidin-2-yl)-6-oxo-1,6-dihydropyrimidin-5-yl]methyl}bipheny 1-2-sulfonamide (yield: 67%) was obtained as a pale yellow oil according to the same reaction and treatment as the Process 3 of Example 1 by using the
5-(4-bromobenzyl)-6-butyl-2-cyclohexyl-3-(5-ethoxypyrimidin -2-yl) pyrimidin-4 (3H)-one obtained in the Process 2 instead of the
5-(4-bromobenzyl)-6-butyl-3-(5-methoxypyrimidin-2-yl)-2-met hylpyrimidin-4(3H)-one in the Process 3 of Example 1.

¹H-NMR(CDCl₃)**δ:**
8.51 (2H, s), 8.14 (1H, dd, J = 7.8, 1.2 Hz), 7.56-7.26 (7H, m), 4.23 (2H, q, J = 6.9 Hz), 3.94 (2H, s), 3.57 (1H, s), 2.65 (2H, t, J = 7.7 Hz), 1.93-1.19 (16H, m), 0.98-0.91 (14H, m).

### Process 4:

4'-{[4-butyl-2-cyclohexyl-1-(5-ethoxypyrimidin-2-yl)-6-oxo-1,6-dihydropyrimidin-5-yl]methyl}biphenyl-2-sulfonami de (yield: 83%) was obtained as a pale yellow oil according to the same reaction and treatment as the Process 4 of Example 1 by using the
N-tert-butyl-4'-{[4-butyl-2-cyclohexyl-1-(5-ethoxypyrimidin -2-yl)-6-oxo-1,6-dihydropyrimidin-5-yl]methyl}biphenyl-2-su lfonamide obtained in the Process 3 instead of the N-tert-butyl-4'-{[4-butyl-1-(5-methoxypyrimidin-2-yl)-2-met hyl-6-oxo-1,6-dihydropyrimidin-5-yl]methyl}biphenyl-2-sulfo namide in the Process 4 of Example 1.

¹H-NMR(CDCl₃)**δ:**
8.50 (2H, s), 8.12 (1H, dd, J = 7.9, 1.4 Hz), 7.56 (1H, td, J = 7.6, 1.2 Hz), 7.46 (1H, td, J = 7.6, 1.2 Hz), 7.38-7.28 (5H, m), 4.27-4.19 (4H, m), 3.93 (2H, s), 2.67 (2H, t, J = 7.7 Hz), 1.93-1.20 (16H, m), 1.06-0.91 (5H, m).

### Process 5:

N-{4'-{[4-butyl-2-cyclohexyl-1-(5-ethoxypyrimidin-2-y 1)-6-oxo-1,6-dihydropyrimidin-5-yl]methyl}biphenyl-2-ylsulf onyl}cyclopropanecarboxamide (yield: 85%) was obtained as a white amorphous according to the Process 5 of Example 1 by using the
4'-{[4-butyl-2-cyclohexyl-1-(5-ethoxypyrimidin-2-yl)-6-oxo-1,6-dihydropyrimidin-5-yl]methyl}biphenyl-2-sulfonamide obtained in the Process 4 instead of the 4'-{[4-butyl-1-(5-methoxypyrimidin-2-yl)-2-methyl-6-oxo-1,6 -dihydropyrimidin-5-yl]methyl}biphenyl-2-sulfonamide and cyclopropanecarbonyl chloride instead of the propionyl chloride in the Process 5 of Example 1.

¹H-NMR(CDCl₃)**δ:**
8.46 (2H, s), 8.23 (1H, d, J = 8.0 Hz), 7.58 (1H, td, J = 7.4, 1.2 Hz), 7.50 (1H, td, J = 7.4, 1.2 Hz), 7.30-7.24 (5H, m), 4.22 (2H, q, J = 6.9 Hz), 3.93 (2H, s), 2.77 (2H, t, J = 7.7 Hz), 1.88-1.30 (16H, m), 1.14-0.95 (6H, m), 0.83-0.78 (2H, m), 0.64-0.57 (2H, m).

### Test example 1: Angiotensin II antagonistic activity in isolated rabbit blood vessels

By using a specimen of isolated rabbit blood vessels, antagonistic activity of the compounds of the invention against angiotensin II type 1 receptor was estimated from a dose-response curve of angiotensin II-induced blood vessel contraction. Specifically, the specimen of thoracic aorta ring of a rabbit (New Zealand White: male, 2.4 to 3.0 kg) was suspended in a magnus bath filled with Krebs-Henseleite buffer (composition: 118 mM NaCl, 4.7 mM KCl, 2.55 mM CaCl₂, 1.18 mM MgSO₄, 1.18 mM KH₂PO₄, 24.88 mM NaHCO₃, and 11.1 mM D-glucose), and angiotensin II (10 nM)-induced contraction was obtained in the presence of the compounds of each example (1 nmol to 10 µmol/L). During the measurement, the inside temperature of the magnus bath was maintained at 37°C and the bath was continuously ventilated with a sufficient amount of mixed gas (95% O₂ and 5% CO₂) . The angiotensin II-induced contraction was converted into a relative value (%) that is based on the angiotensin II (10 nM)-induced contraction in the absence of the compounds of each example. From the concentration-response curve obtained therefrom, 50% inhibition concentration (IC₅₀ value) was calculated by using SAS Preclinical Package Ver 5.0 (trade name, manufactured by SAS institute Japan Co., Tokyo, Japan), which is a statistical analysis program.

As a result, it was found that the compound described in each example has an angiotensin II inhibition activity at 10 µM concentration. The inhibitory activities (%) in comparison to those at 0.1 µM of these compounds are shown in Table 1. As shown in Table 1, it was confirmed that the compound of the present invention has potent angiotensin II antagonistic activity, which is equivalent to that of telmisartan. Under the same condition, the angiotensin II activity inhibition rate of telmisartan was 85.3%.

**[Table 1]**

| Example No. | Angiotensin II activity inhibition rate (%) at 0.1µM concentration |
|---|---|
| 1 | 71.7 |
| 2 | 91.9 |
| 3 | 90.0 |
| 4 | 100 |
| 5 | 92.4 |
| 7 | 89.3 |
| 8 | 77.5 |
| 9 | 100 |
| 10 | 81.9 |

### Test example 2: PPARγ activation activity

The agonistic activity of the compounds of the invention on PPARγ was measured based on the transfection assay using COS7 cells (DS Pharma Biomedical Co., Ltd., Osaka, Japan), which are the cell line derived from the kidney of the African green monkey. COS7 cells were cultured under 5% CO₂ concentration, and DMEM medium containing 10% fetal bovine serum, glutamic acid, and antibiotics was used as a medium.

As an expression vector, a chimera in which DNA binding domain of Ga14, which is a yeast transcription factor, and ligand binding domain of human PPARγ2 are fused, i.e., a fused product between the amino acids 1 to 147 of Ga14 transcription factor and the amino acids 182 to 505 of human PPARγ2, was used. Furthermore, as a reporter vector, a firefly luciferase containing five copies of Ga14 recognition sequence in the promoter region was used. Plasmid transfection to the cells was performed according to a method which uses jetPEI (trade name, manufactured by Funakoshi Co., Ltd., Tokyo, Japan). Furthermore, β-galactosidase expression vector was employed as an internal standard.

After the transfection of the cells, the medium was replaced with a DMEM medium (containing 1% serum) added with the compound, and the cells were further cultured for 16 hours. After that, the luciferase activity and β-galactosidase activity in the cell lysis solution were measured.

For the present test, dimethyl sulfoxide (DMSO) was used for dissolution and dilution of the test compounds, and during the cell treatment, the DMSO concentration in DMEM medium (containing 1% serum) was adjusted to 0.1%. As a positive compound, rosiglitazone (trade name, manufactured by ALEXIS Corporation, Switzerland) was used. The luciferase activity (%) of the test compounds (1 to 30 µmol/L) was calculated when the luciferase activity of rosiglitazone (3 to 10 µmol/L) is 100% and the luciferase activity in the absence of the test compound is 0%. The 50% effective concentration of the test compound (EC₅₀, 50% effect concentration) was calculated by using SAS Preclinical Package Ver 5. 0 (trade name, manufactured by SAS institute Japan Co., Tokyo, Japan), which is a statistical analysis program.

The results of representative compounds are shown in Table 2. As shown in Table 2, it was confirmed that the compounds of the invention have a potent PPARγ activation activity. Under the same condition, the PPARγ activation activity of telmisartan, i.e., EC₅₀, was 1 to 5 µM. In addition, it was confirmed that compounds of Examples not shown in Table 2 also have PPARγ activation activity at a concentration of 30 µM.

**[Table 2]**

| Example No. | EC₅₀ (µM) |
|---|---|
| 7 | 2.21 |
| 8 | 3.69 |
| 9 | 3.52 |
| 11 | 3.37 |
| 13 | 2.79 |
| 14 | 1.36 |
| 15 | 1.05 |
| 16 | 2.35 |
| 17 | 0.96 |
| 18 | 0.71 |
| 19 | 0.51 |

From the results obtained above, it was confirmed that the compounds represented by the general formula (I) have both a potent angiotensin II receptor antagonistic activity and a PPARγ activation activity. Thus, it was found that the compounds represented by the formula (I) and pharmaceutically acceptable salts thereof are useful as an effective component of a prophylactic and/or therapeutic agent for disorders involved with angiotensin II and PPARγ, for example, hypertension, heart diseases, angina pectoris, cerebrovascular disorders, cerebral circulatory disorders, ischemic peripheral circulatory disorders, renal diseases, arteriosclerosis, inflammatory diseases, type 2 diabetes, diabetic complications, insulin resistance syndrome, syndrome X, metabolic syndrome, and hyperinsulinemia.

### INDUSTRIAL APPLICABILITY

The invention provides a novel compound of sulfonamide derivative represented by the formula (I) of the invention, or salt or a solvate thereof, which has both an angiotensin II receptor antagonistic activity and a PPARγ activation activity. They can be used as an effective component of a novel pharmaceutical product, i.e., a prophylactic and/or therapeutic agent for disorders involved with angiotensin II and PPARγ, for example, hypertension, heart diseases, angina pectoris, cerebrovascular disorders, cerebral circulatory disorders, ischemic peripheral circulatory disorders, renal diseases, arteriosclerosis, inflammatory diseases, type 2 diabetes, diabetic complications, insulin resistance syndrome, syndrome X, metabolic syndrome, and hyperinsulinemia, and therefore have an industrial applicability.

## Claims

1. A sulfonamide derivative represented by the formula (I) below or a salt thereof, or a solvate thereof: 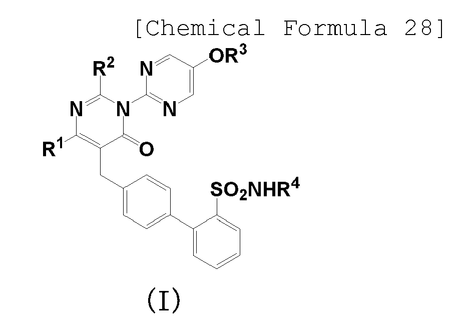 [in the formula, R¹ represents a C₁₋₆ alkyl group,
R² represents a C₁₋₆ alkyl group, a C₃₋₈ cycloalkyl group, or a C₁₋₆ alkyl-pyridinyl-C₁₋₆ alkyl group,
R³ represents a hydrogen atom, a C₁₋₆ alkyl group, a halo C₁₋₆ alkyl group, a C₃₋₈ cycloalkyl group, or a C₁₋₆ alkoxy-C₁₋₆ alkyl group, and
R⁴ represents a hydrogen atom, a C₁₋₆ alkyl group, a C₁₋₆ alkanoyl group or a C₃₋₈ cycloalkylcarbonyl group.

2. The sulfonamide derivative described in Claim 1 or salt thereof, or solvate thereof, in which the compound represented by the formula (I) is a compound selected from a group consisting of:
N-{4'-{[4-butyl-1-(5-methoxypyrimidin-2-yl)-2-methyl-6-oxo-1,6-dihydropyrimidin-5-yl]methyl}biphenyl-2-ylsulfony l}propionamide,
N-{4'-{[4-butyl-1-(5-methoxypyrimidin-2-yl)-2-methyl-6-oxo-1,6-dihydropyrimidin-5-yl]methyl}biphenyl-2-ylsulfony l}hexanamide,
N-{4'-{[4-butyl-1-(5-methoxypyrimidin-2-yl)-2-methyl-6-oxo-1,6-dihydropyrimidin-5-yl]methyl}biphenyl-2-ylsulfony 1}cyclopropanecarboxamide,
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclopentanecarboxamide,
N-{4'-{[4-butyl-1-(5-methoxypyrimidin-2-yl)-2-methyl-6-oxo-1,6-dihydropyrimidin-5-yl]methyl}biphenyl-2-ylsulfony l}cyclohexanecarboxamide,
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }propionamide,
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }hexanamide,
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopropanecarboxamide,
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclopentanecarboxamide,
N-{4'-{[4-butyl-1-(5-ethoxypyrimidin-2-yl)-2-methyl-6 -oxo-1,6-dihydropyrimidin-5-yl]methyl}biphenyl-2-ylsulfonyl }cyclohexanecarboxamide,
N-{4'-{{4-butyl-1-[5-(difluoromethoxy)pyrimidin-2-yl] -2-methyl-6-oxo-1,6-dihydropyrimidin-5-yl}methyl}biphenyl-2 -ylsulfonyl}cyclopropanecarboxamide,
N-{4'-{{4-butyl-2-methyl-6-oxo-1-[5-(2,2,2-trifluoroe thoxy)pyrimidin-2-yl]-1,6-dihydropyrimidin-5-yl}methyl}biph enyl-2-ylsulfonyl}cyclopropanecarboxamide,
N-{4'-{[4-butyl-1-(5-ethoxypyrimidin-2-yl)-2-ethyl-6-oxo-1,6-dihydropyrimidin-5-yl]methyl}biphenyl-2-ylsulfonyl} cyclopropanecarboxamide,
N-{4'-{[4-butyl-1-(5-ethoxypyrimidin-2-yl)-2-isopropy l-6-oxo-1,6-dihydropyrimidin-5-yl]methyl}biphenyl-2-ylsulfo nyl}cyclopropanecarboxamide,
N-{4'-{[4-butyl-2-cyclopropyl-1-(5-methoxypyrimidin-2 -yl)-6-oxo-1,6-dihydropyrimidin-5-yl]methyl}biphenyl-2-ylsu lfonyl}cyclopropanecarboxamide,
N-{4'-{[4-butyl-2-cyclopropyl-1-(5-ethoxypyrimidin-2-yl)-6-oxo-1,6-dihydropyrimidin-5-yl]methyl}biphenyl-2-ylsul fonyl}cyclopropanecarboxamide,
N-{4'-{[4-butyl-2-cyclobutyl-1-(5-ethoxypyrimidin-2-y l)-6-oxo-1,6-dihydropyrimidin-5-yl]methyl}biphenyl-2-ylsulf onyl}cyclopropanecarboxamide,
N-{4'-{[4-butyl-2-cyclopentyl-1-(5-ethoxypyrimidin-2-yl)-6-oxo-1,6-dihydropyrimidin-5-yl]methyl}biphenyl-2-ylsul fonyl}cyclopropanecarboxamide, and
N-{4'-{[4-butyl-2-cyclohexyl-1-(5-ethoxypyrimidin-2-y l)-6-oxo-1,6-dihydropyrimidin-5-yl]methyl}biphenyl-2-ylsulf onyl}cyclopropanecarboxamide.

3. A pharmaceutical composition comprising the sulfonamide derivative described in Claim 1 or 2, or salt thereof, or solvate thereof, and a pharmaceutically acceptable carrier.

4. A pharmaceutical composition which has both angiotensin II receptor antagonistic activity and PPARγ activation activity and comprises as an effective component the sulfonamide derivative described in Claim 1 or 2 or salt thereof, or solvate thereof.

5. An agent for preventing and/or treating a circulatory disorder which comprises as an effective component the sulfonamide derivative described in Claim 1 or 2 or salt thereof, or solvate thereof.

6. The agent for preventing and/or treating a circulatory disorder according to Claim 5, wherein the circulatory disorder is hypertension, heart diseases, angina pectoris, cerebrovascular disorders, cerebral circulatory disorders, ischemic peripheral circulatory disorders, renal diseases, or arteriosclerosis.

7. An agent for preventing and/or treating a metabolic disorder which comprises as an effective component the sulfonamide derivative described in Claim 1 or 2 or salt thereof, or solvate thereof.

8. The agent for preventing and/or treating a metabolic disorder according to Claim 7, wherein the metabolic disorder is type 2 diabetes, diabetic retinopathy, diabetic neuropathy, diabetic nephropathy, insulin resistance syndrome, metabolic syndrome, or hyperinsulinemia.

9. A method of preventing and/or treating a circulatory disorder, **characterized in that** an effective amount of the sulfonamide derivative described in Claim 1 or 2 or salt thereof, or solvate thereof is administered to a patient who is in need of treatment.

10. A method of preventing and/or treating a metabolic disorder, **characterized in that** an effective amount of the sulfonamide derivative described in Claim 1 or 2 or salt thereof, or solvate thereof is administered to a patient who is in need of treatment.

11. Use of the sulfonamide derivative described in Claim 1 or 2 or salt thereof, or solvate thereof for producing a preparation for preventing and/or treating a circulatory disorder.

12. Use of the sulfonamide derivative described in Claim 1 or 2 or salt thereof, or solvate thereof for producing a preparation for preventing and/or treating a metabolic disorder.

13. The sulfonamide derivative described in Claim 1 or 2 or salt thereof, or solvate thereof as a prophylactic and/or therapeutic agent that has both angiotensin II receptor antagonistic activity and PPARγ activation activity.
